(19) Europäisches Patentamt
European Patent Office
Office européen des brevets

(11) Numéro de publication: **0 219 400**
**B1**

(12) **FASCICULE DE BREVET EUROPEEN**

(45) Date de publication du fascicule du brevet: **08.08.90**

(21) Numéro de dépôt: **86402085.4**

(22) Date de dépôt: **23.09.86**

(51) Int. Cl.⁵: **A 61 K 37/02,** C 12 P 21/00, C 07 K 3/12

(54) **Glycopeptides et glycoprotéines, toute composition les contenant leur procédé de préparation et leurs applications anticoagulantes.**

(30) Priorité: **23.09.85 FR 8514083**

(43) Date de publication de la demande: **22.04.87 Bulletin 87/17**

(45) Mention de la délivrance du brevet: **08.08.90 Bulletin 90/32**

(84) Etats contractants désignés: **AT BE CH DE FR GB IT LI LU NL SE**

(56) Documents cités:
CHEMICAL ABSTRACTS, vol. 102, no. 3, 21 janvier 1985, page 377, résumé 20980x, Columbus, Ohio, US; J-M. NICAUD et al.: "Mutants of Myxococcusin protein secretion: an approach to study of a secretory mechanism", & APPL. MICROBIOL. BIOTECHNOL. 1984, 20(5), 344-50

(73) Titulaire: **UNIVERSITE DE TECHNOLOGIE DE COMPIEGNE**
**Centre Benjamin Franklin Rue Roger Couttolenc - BP 233**
**F-60206 Compiègne Cédex (FR)**

(72) Inventeur: **El Akoum, Ali**
**1, Square Philibert Delorme Appt. 814**
**F-60200 Compiegne (FR)**
Inventeur: **Guespin-Michel, Janine**
**7, rue du Mal Douglas Haig**
**F-60200 Compiegne (FR)**
Inventeur: **Sigot, Michel**
**11, avenue de la Division Leclerc**
**F-60200 Compiegne (FR)**

(74) Mandataire: **Peaucelle, Chantal et al**
**S.C. Ernest Gutmann - Yves Plasseraud 67, boulevard Haussmann**
**F-75008 Paris (FR)**

Courier Press, Leamington Spa, England.

**EP 0 219 400 B1**

(56) Documents cités:

CHEMICAL ABSTRACTS, vol. 105, no. 5, 4 aout 1986, page 188, résumé no. 36763b, Columbus, Ohio, US; A.M. BRETON et al.: "Myxococcus xanthus, a gram negative non pathogenic bacterium, that secretes proteins into the extracellular growth medium, is a potential cloning host for excreted proteins production", & EUR. CONGR. BIOTECHNOL. 3rd 1984, 3, 441-6

APPL. MICROBIOL. BIOTECHNOL., vol. 19, no. 1, 1984, pages 67-69; G. YOUNES et al.: "Enhancement of extracellular enzymatic activities produced by immobilized growing cells of Myxococcus xanthus"

ARCH. MICROBIOL., vol. 116, no. 1, 1978, pages 51-59, Springer-Verlag; G. GNOSSPELIUS: "Myxobacterial slime and proteolytic activity"

PHYSIOL. PLANT., vol. 28, no. 3, 1973, pages 523-529; S. STAHL: "Slime of Myxococcus virescens"

## Description

L'invention est relative à des molécules glycopeptidiques et glycoprotéiniques présentant des propriétés biologiques leur permettant notamment de jouer un rôle régulateur vis-à-vis de la coagulation sanguine.

L'invention vise également toute composition contenant ces molécules glycopeptidiques et glycoprotéiniques, ainsi que toute composition contenant ces molécules glycopeptidiques et glycoprotéiniques et possédant des propriété biologiques leur permettant de jouer un rôle vis-à-vis de la coagulation sanguine.

De telles molécules glycopeptidiques et glycoprotéiniques peuvent être obtenues notamment à partir des myxobactéries qui produisent un mucus qui absorbe le rouge congo.

On sait que l'héparine est sans doute à ce jour l'un des médicaments le plus largement utilisé dans l'application anticoagulante. Elle est en effet capable d'intervenir à plusieurs niveaux dans les cascades de réactions enzymatiques successives, qui sont normalement mises en jeu au cours de l'hémostase physiologique, dans toute situation susceptible d'entraîner une hypercoagulabilité du sang. Elle es plus particulièrement capable de déprimer simultanément un grande nombre des facteurs de la coagulation intervenant dans la création et le maintient des différentes formes d'hypercoagulabilité.

C'est évidemment pour pallier les effets d'hypercoagulabilité que l'on a couramment recours aux puissantes propriétés anticoagulantes de l'héparine, en vue de remaner le mécanisme coagulation-fibrinolyse à l'équilibre, chaque fois que celui-ci subit une perturbation importante, par exemple à l'occasion d'une intervention chirurgicale sur l'hôte. Il est cependant bien connu que ces tentatives de rééquilibrage sont extrêmement délicates et que, en conséquence, l'administration de doses trop élevées de médicament anticoagulation—ou l'insuffisante sélectivité de celui-ci—dans le but de prévenir les risques d'hypercoagulation, par exemple l'apparition de thromboses post-chirurgicales, peut finalement être à l'origine d'hémorragies graves: d'où la nécessité d'une surveillance constante des patients traités et des ajustements nécessaires des doses administrées—en continu ou en discontinu—en fonction des résultats de tests, notamment de coagulabilité globale, comme le temps de Howel, qui doivent être pratiqués à intervalles réguliers.

On rappellera ci-après, dans les limites de ce qui est nécessaire pour la clarté de l'exposé, quelques unes des notions de base, et volontairement simplifiées, relatives à la coagulation. Le processus de coagulation comprend en effet trois phases généralement décrites comme successives, même si elles sont étroitement intriquées:

la thromboplastinoformation, phase de formation de prothrombinase (ou thromboplastine active);

la thrombinoformation, phase qui peut se résumer à la transformation de la prothrombine en thrombine sous l'influence de la prothrombinase en présence de calcium ionisé et enfin;

la fibrinoformation, phase au cours de laquelle le fibrinogène sanguin est, sous l'effet de la thrombine, transformé en fibrine, protéine qui tend à devenir insoluble.

La formation de prothrombinase se fait, au cours de l'étape de thromboplastinoformation, essentiellement selon deux voies différentes: la voie intrinsèque ou endogène, et la voie extrinsèque ou exogène, lesquelles aboutissent à la formation de prothrombinases d'origines respectivement plasmatique et tissulaire, toutes deux susceptibles d'activer la prothrombine en thrombine active.

La voie (ou système) intrinsèque ou endogène fait intervenir un grand nombre de facteurs ou proenzymes plasmatiques susceptibles d'être successivement activés (facteurs XII, XI, IX, VIII et X), où chaque produit activé (facteurs XIIa, XIa, IXa, VIIIa et Xa) agit comme une enzyme capable d'activer la proenzyme suivante, le facteur X activé (Xa) intervenant alors, notamment par réaction avec le facteur V et un phospholipide d'origine plaquettaire, dans la production de prothrombinase plasmatique endogène active. Le système extrinsèque ou exogène, qui peut notamment se trouver sous la dépendance directe d'une lésion tissulaire, fait appel à un nombre plus limité de facteurs et comporte notamment la production de thromboplastine tissulaire qui, en combinaison avec le facteur VII, peut, tout comme le facteur VIIIa, transformer le facteur X inactif en facteur Xa. La séquence d'activation de la prothrombine en thrombine est ensuite sensiblement la même que pour le système intrinsèque, mais le phospholipide est ici d'origine tissulaire et non plasmatique.

On peut donc, à la limite, exprimer l'idée que les deux voies, intrinsèque et extrinsèque, se rejoignent au niveau de l'activation du facteur X (aussi appelé facteur Stuart), les deux phases suivantes de la coagulation—thrombinoformation et fibrinoformation—ne donnant alors plus lieu à une distinction entre voies intrinsèque et extrinsèque.

L'aboutissement du processus de coagulation consiste dans la formation d'un caillot de fibrine insoluble, destiné notamment à colmater la lésion à l'origine du déclenchement de ce processus, par exemple au niveau d'un vaisseau sanguin.

Ces processus de coagulation font normalement place ensuite à un processus, dénommé fibrinolyse, destiné à produire la lyse du caillot, notamment sous l'effet de la plasmine, enzyme qui n'existe normalement dans le sang circulant que sous la forme d'un précurseur inactif, le plasminogène, la fibrine elle-même constituant néanmoins l'un des facteurs susceptibles de déclencher la transformation du plasminogène inactif en plasmine fibrinolytiquement active.

En fait, bien que l'on ait, dans ce qui précède, présenté les systèmes de coagulation et de fibrinolyse comme deux processus se produisant successivement dans le temps, il n'en est pas normalement toujours ainsi dans la réalité. En fait, il s'agit de mécanismes équilibrés, selon des processus extrêmement complexes, sous la dépendance de facteurs activateurs et inhibiteurs harmonieusement opposés. Le déséquilibrage de ces mécanismes, dans le sens d'une hypercoagulabilité, est alors susceptible d'entraîner des thromboses. A l'opposé, un déséquilibre dans le sens d'une hypocoagulabilité, expose l'hôte à des risques hémorragiques.

En ce qui concerne les myxobactéries qui peuvent être utilisées pour la préparation des glycopeptides selon l'invention ou de toute composition les contenant, on rappellera ci-après les éléments suivants. L'ordre des myxobactérales (myxobactéries) est très homogène. Des études sur l'ordre des myxobactéries ainsi que sur la toxonomie des myxobactéries ont été effectuées par Reichenbach H. et Dworkin M., The Order Myxobacterales in the prokaryotes, a handbook on habitats, isolation and identification of bacteria, 20, p. 328—355, D. McCurdy, H., Canadian Journal of Microbiology, Vol. 15, 1969, p. 1 453—1 461.

L'ordre des myxobactéries est constitué de bactéries à Gram négatif, unicellulaires, aérobies strictes, mobiles par glissement et capables de former des fructifications contenant des formes de résistance ou myxospores. Elles vivent au détriment de macromolécules en excrétant de nombreux enzymes lytiques, ainsi que des antibiotiques et un mucus. Leur ADN présente une grande homogénéité de composition (66 à 72% G+C).

Les critères taxonomiques permettant de différencier les deux sous-ordres, quatre familles et douze genres qui les composent sont la forme des bactéries, des fructifications et des myxospores, leur composition en acides gras et la nature de leur mucus.

On distingue deux sous-ordres d'après ce dernier critère: les Cystobacterinae et les Sorangiacae. Les Cystobacterinae produisent un mucus qui absorbe le rouge Congo alors que le mucus produit par les Sorangiacae ne l'absorbe pas. La famille des Myxococcacae à laquelle appartient l'espèce très connue Myxococcus xanthus fait parties des Cystobacterinae (Studies on the taxonomy of the Myxobacterials I. Record of Canadian isolates and survey of methods, H. McCurdy).

L'homogénéité des propriétés des mucus excrétés par les différentes espèces de Cystobacterinae est aussi soulignée par les expériences de Burchard (Burchard R. P., 1982, Trails following by gliding bacteria, Journal of Bacteriology, p. 943—950) montrant que la trace muqueuse laissée par une espèce peut être semblable à celle de bactéries d'autres espèces ou genres voisins, de ce sous-ordre.

Compte tenu de l'homogénéité de la composition et des propriétés du mucus excrété par les espèces du sous-ordre des Cystobacterinae, les résultats concernant une souche appartenant au sous-ordre des Cystobacterinae peuvent être considérés, à ce jour, comme valables pour toutes les espèces du sous-ordre des Cystobacterinae (Myxococcus, Corallococcus, Angiococcus, Archangium, Cystobacter, Melihangium, Stigmatela) ainsi que pour certains mutants de l'une de ces souches sauvages.

Les myxobactéries ont fait l'objet de nombreuses publications, notamment relativement à leur secrétion abondante qui contient des antibiotiques, des protéines et un mucus polysaccharidique (cf. J. W. Sutherland and S. Thomson, Journal of General Microbiology, 1975, 89, 124—132) comparisons of polysaccharides produced by Myxococcus strains).

Mais, compte tenu de la complexité de cette secrétion, on n'a encore à ce jour jamais isolé d'extraits de cette secrétion, même à fortiori mis en évidence de propriétés biologiques particulières.

Or, la Société Demanderesse a trouvé de façon inattendue en étudiant les secrétions du milieu de culture de myxobactéries dont le mucus absorbe le rouge Congo, des molécules glycopeptidiques et glycoprotéiniques possédant une activité biologique, notamment vis-à-vis de la coagulation sanguine.

L'invention a pour but de fournir des principes actifs de médicaments (et les médicaments eux-mêmes) qui permettent de remédier au moins en partie aux difficultés énoncées à propos de l'héparine, notamment qui soient capables de permettre un rééquilibrage éventuel et/ou un contrôle plus aisé, au prix d'une moindre surveillance clinique du système coagulation-fibrinolyse chez des patients affectés d'une pathologie de la coagulation ou ayant subi un traitement, tel qu'une intervention chirurgicale, qui les exposent à des risques d'hypercoagulabilité.

L'invention concerne plus particulièrement des glycopeptides et glycoprotéines, et toute composition les contenant, exerçant un effet régulateur à l'égard de la coagulation, notamment dans le sens d'un retard à la coagulation, cependant par la mise en jeu d'actions inhibitrices différentes de celles de l'héparine, voire plus sélectives que celles de l'héparine.

L'invention a pour objet de fournir des glycopeptides et des glycoprotéines, ou toute composition les contenant, présentent une action anticoagulante caractérisée essentiellement par une inhibition directe de la thrombine, sans impact négatif sur la structure du fibrinogène et sa capacité de se polymériser.

L'invention a également pour objet de fournir des glycopeptides et des glycoprotéines ou toute composition les contenant, présentant des propriétés anticoagulantes et d'innocuité vis-à-vis des cellules endothéliales.

L'invention a donc pour objet de fournir un principe actif particulièrement intéresssant vis-à-vis des propriétés anticoagulantes dont les propriétés sont stables et qui n'est pas toxique.

L'invention a également pour objet de fournir des principes actifs anticoagulants qui peuvent être obtenus à l'aide de produits extrêmement courants et peu onéreux.

L'invention a pour objet de fournir des glycopeptides et des glycoprotéines et toute composition les contenant présentant des propriétés anticoagulantes et qui peuvent être produits en masse, de façon

EP 0 219 400 B1

simple et rentable à l'échelle industrielle.

L'invention a pour objet des glycopeptides et glycoprotéines présents dans le mucus excrété par les Myxobactéries Cystobacterinae en dans le milieu de culture des Myxobactéries Cystobacterinae caractérisés par les propriétés suivantes:

leur poids moléculaire est compris d'environ 5 000 à environ 20 000 daltons, notamment d'environ 5 000 à environ 10 000;

les motifs contenus sont des hexoses et des hexoses aminés, appartenant au groupe constitué par le mannose, le glucose, le galactose, la galactosamine, la glucosamine;

leur comportement en chromatographie HPLC n'est pas modifié par le sodium dodécylsulfate;

leur pH isoélectrique est d'environ 3 à environ 5, notamment environ 3,6;

ils sont anticoagulants sur sang total à partir d'une concentration d'environ 80 μg/ml;

leur activité anticoagulante est d'environ 2 à environ 10 UI/mg;

ils entraînent un allongement du temps de céphaline-kaolin;

ils entraînent un allongement du temps de Quick;

ils entraînent un allongement du temps de thrombine;

ils inactivent le facteur Xa.

Plus particulièrement, les glycopeptides et les glycoprotéines selon l'invention ont la composition suivante:

70—80% (en poids) de résidus d'acides aminés;

30—20% (en poids) de motifs sucres, lesquels motifs sucres sont constitués par 60 à 65% (en poids de sucres neutres et 35 à 40% (en poids) d'osamine.

La composition des motifs de sucres neutres est constituée par 30 à 35% (en poids) de glucose, 15 à 20% (en poids) de mannose, 10 à 15% (en poids) de galactose.

La composition des osamines comprend de 20 à 25% (en poids) de glucosamine et de 10 à 15% (en poids) de galactosamine.

Selon un mode de réalisation avantageux, la composition en sucres des glycopeptides et glycoprotéines de l'invention est la suivante,

environ 35% (en poids) de glucose,

environ 11,4% (en poids) de galactose,

environ 16,3% (en poids) de mannose,

environ 23,3% (en poids) de glucosamine et

environ 14% (en poids) de galactosamine.

Selon un mode de réalisation avantageux, la composition des glycopeptides et glycoprotéines de l'invention, en acides aminés est la suivante

30 à 35% (en poids) d'acide glutamique;

25 à 30% (en poids) de sérine;

5 à 10% (en poids) d'acide aspartique;

5 à 10% (en poids) de glycine;

5 à 10% (en poids) d'isoleucine;

5 à 10% (en poids) d'alanine;

0 à 5% (en poids) de valine;

0 à 5% (en poids) de leucine;

0 à 5% (en poids) de thréonine;

0 à 5% (en poids de tyrosine;

moins de 2% (en poids) de proline;

moins de 2% (en poids) de lysine;

moins de 2% (en poids) de phénylalanine;

moins de 2% (en poids) d'histidine;

moins de 2% (en poids) d'arginine.

Selon un autre mode de réalisation de l'invention, la composition des glycopeptides et glycoprotéines de l'invention en acides aminés est la suivante

environ 31% (en poids) d'acide glutamique,

environ 28% (en poids) de sérine,

environ 7% (en poids) d'acide aspartique,

environ 6% (en poids) de glycine,

environ 5,5% (en poids) d'isoleucine,

environ 5% (en poids) d'alanine,

environ 3% (en poids) de valine,

environ 3% de (en poids) leucine,

environ 2,5% (en poids) de thréonine,

environ 2,5% (en poids) de tyrosine,

moins d'environ 2% (en poids) de proline,

moins d'environ 2% (en poids) de phénylalanine,

moins d'environ 2% (en poids) d'histidine,

moins d'environ 2% (en poids) d'arginine.

5

Les glycopeptides et glycoprotéines selon l'invention peuvent présenter également les caractéristiques suivantes:

un titre anti-IIa est d'environ 8,5 UI/mg,

un titre anti-Xa est d'environ 180 UI/mg.

Pour vérifier le poids moléculaire, on peut avoir recours à l'électrophorèse en utilisant un kit ou coffret de calibration.

Pour vérifier la composition des glycopeptides et glycoprotéines selon l'invention, on peut par exemple avoir recours aux méthodes suivantes.

La composition globale en acides aminés peut être déterminée par une méthode classique, telle que la méthode de Lowry, Oh. H., Rosebrough N. J., Farr A. L., Randal R. J. dans J. Biol. Chem., 193, 265—273, 1951.

La composition globale en sucres de ces molécules peut être déterminée par une méthode classiquement utilisée dans laquelle on a recours à l'anthrone comme réactif, telle qu'elle est décrite notamment dans "Determination of dextran with anthrone", Analyt. Chem., 25, p. 1 656—1 681, Scott T. A. and Melvin, 1953.

Le réactif utilisé dans cette méthode, à savoir l'anthrone, est particulièrement sensible aux hexoses.

Le pH isoélectrique peut être déterminé par électrofocalisation telle qu'elle est décrite dans Anders Winter, Krishna Ek and Ulla-Birgitta Anderson: Analytical Electrofocusing in thin layers of polyacrylamide gels, LKB Application note 250, 1977.

L'homogénéité peut également être déterminée par électrofocalisation selon la méthode dont la référence est mentionnée ci-dessus.

En ce qui concerne le temps de thrombine (temps anti-IIa), qui est un type de mesure reflétant plutôt l'action des glycopeptides sur l'inhibition du facteur IIa, on se reportera aux ouvrages suivants: Cazenave, Introduction à l'étude de l'hémostase et de la thrombose, 1982; Caen J., Larieu N. J. et Samama N., L'hémostase, méthodes d'exploration diagnostiques pratiques, 1980.

En ce qui concerne le temps de céphaline-kaolin qui est un type de mesure reflétant plutôt l'action des glycopeptides et des glycoprotéines au niveau de la voie endogène, on se reportera également aux ouvrages cités ci-dessus.

En ce qui concerne le temps de Quick et le temps anti-Xa, on se reportera aux ouvrages cités ci-dessus.

L'invention concerne également un procédé, pour obtenir de tels glycopeptides et glycoprotéines, ce procédé étant caractérisé par:

la séparation et la récupération du surnageant provenant d'un milieu de culture de myxobactéries du sous-ordre des Cystobacterinae ou de mutants de celles-ci;

l'élimination à partir du surnageant des substances de poids moléculaires inférieurs à environ 1 000 daltons;

l'élimination à partir du surnageant des substances insolubles dans l'eau;

et la récupération de la fraction du surnageant débarrassée des susbtances de poids moléculaires inférieure à environ 1 000 daltons et débarrassée des substances insolubles dans l'eau;

la purification de cette fraction par chromatographie sur colonne de pseudo-affinité;

la purification subséquente de la susdite fraction par chromatographie sur colonne de gel de polymère réticulé de dextrane.

La matière première à partir de laquelle les glycopeptides de l'invention peuvent être obtenus peut être constituée par le surnageant provenant d'un milieu de culture de myxobactéries du sous-ordre des Cystobacterinae ou de mutants de celles-ci, les Cystobacterinae étant caractérisées par le fait qu'elles produisent un mucus qui absorbe le rouge Congo.

Le milieu de culture de ces Cystobacterinae peut être constitué par un milieu solide ou liquide.

On a de préférence recours à un milieu de culture liquide.

La souche de myxobactéries utilisée est avantageusement constituée par une souche de *Myxococcus xanthus*.

La souche utilisée est par exemple la *Myxococcus xanthus* CM011, qui dérive de la souche I448, par l'insertion (No. 11) d'un transposon Tn5, selon la technique classique décrite dans "Introduction of transposon Tn5 into Myxococcus for analysis of developmental and other non selectable mutants", Proc. Natl. Acad. Sci. USA, 78, 425—429. La sourche I448 a fait l'objet d'un dépôt le 3 mai 1985, à la Collection Nationale de Cultures de Micro-organismes (CNCM), 28 rue du Docteur Roux, 75015 Paris (France).

Cette insertion confère à la souche notamment la capacité de produire 50% de plus de sucres dans le milieu que la souche d'origine.

A titre d'exemple, on peut également utiliser la souche DK1622, telle que décrite par D. Kaiser dans Proc. Natl. Acad. Sci. USA, 76, 5 952—5 956, 1979.

Une telle souche peut être cultivée en milieu liquide, agité, constitué de Bactocasitone tamponnée commercialisée par la Société Difco.

En ce qui concerne la séparation du surnageant, on prélève celui-ci de préférence au début de la phase stationnaire de croissance des myxobactéries, en d'autres termes, à la fin de la phase exponentielle de croissance.

Les phases d'élimination des substances de poids moléculaires inférieurs à environ 1 000 daltons, ainsi que la phase d'élimination des substances insolubles peuvent être inversées.

Selon un mode de réalisation préféré du procédé de l'invention, on élimine d'abord les substances de poids moléculaires inférieurs à environ 1 000 daltons.

Pour éliminer les substances de poids moléculaires inférieurs à environ 1 000 daltons, on peut avoir recours à de l'alcool éthylique pur.

L'addition d'alcool conduit à l'obtention d'un précipité qui contient les substances de poids moléculaires supérieurs à environ 1 000 daltons, notamment les glycopeptides selon l'invention, tandis que les substances de poids moléculaires inférieurs à environ 1 000 daltons, passent dans l'alcool.

La phase de traitement à l'alcool a également pour but de concentrer le surnageant débarrassé des substances de poids moléculaires inférieurs à 1 000 daltons.

La phase de traitement à l'alcool peut être effectuée plusieurs fois, si l'on souhaite concentrer davantage.

De façon avantageuse, on traite le surnageant deux fois à l'alcool, ce qui signifie qu'après avoir obtenu un premier précipité à l'alcool, on le resolubilise dans l'eau, la phase aqueuse obtenue étant retraitée par une nouvelle addition d'alcool.

On peut ensuite laver le précipité obtenu ci-dessus à l'acétone, pour délipider partiellement, puis à l'éther, notamment pour sécher.

Après le traitement à l'acétone et à l'éther, on laisse avantageusement sécher le précipité.

La phase d'élimination des substances insolubles dans l'eau peut avoir lieu en suspendant le précipité séché dans de l'eau distillée, puis en effectuant une centrifugation.

Par substances insolubles, on désigne dans ce qui précède et dans ce qui suit, les substances faiblement solubles en milieu aqueux, et qui deviennent insolubles au cours de différentes étapes de procédé, en raison des phases de concentration.

On peut ensuite laver les substances insolubles avec de l'eau distillée et recentrifuger.

Puis, on a avantageusement recours à un traitement de purification pour éliminer les traces d'alcool, d'acétone et d'éther éventuellement utilisés dans les étapes précédentes.

Ce traitement de purification est avantageusement constitué par une ultrafiltration.

Cette étape de purification est avantageusement suivie d'une phase de concentration, par exemple sur membrane.

Le produit obtenu à l'issue de cette étape de concentration peut être conservé après lyophilisation ou évaporation sous vide.

La phase d'élimination des substances insolubles peut avoir lieu à un moment quelconque entre la séparation du surnageant et la purification par chromatographie de pseudo-affinité, et a lieu de préférence après le séchage qui suit le lavage à l'acétone et à l'éther.

La phase d'élimination des substances insolubles peut également avoir lieu après l'obtention du lyophilisat, lequel est dissous dans l'eau, et centrifugé pour éliminer les substances insolubles.

En ce qui concerne la chromatographie de pseudoaffinité, elle désigne une chromatographie dans laquelle n'intervient pas seulement l'affinité, mais aussi la charge, et l'hydrophobicité des éléments chromatographiés (cf. L. Amourache et T. A. Vijayalakshmi, AFfinity chromatography of kid chimosin on histidyl-sepharose, Journal of Chromatography, 303, 285—290, 1984).

On effectue avantageusement cette chromatographie de pseudo-affinité sur une colonne d'histidyl-(sépharose 4B). On utilise un tampon acétate à un pH d'environ 5,5. Cette chromatographie donne plusieurs fractions. On recueille la fraction présentant des propriétés anticoagulantes, telles que mesurées selon le test de coagulation sur sang total décrit ci-après.

On passe ensuite la fraction anticoagulante sur une colonne de gel de polymère réticulé de dextrane, tel que celui commercialisé sous le nom Sephadex® G-25 par la Société Pharmacia Fine Chemicals.

Cette chromatographie permet notamment de se débarrasser du NaCl utilisé dans la chromatographie de pseudo-affinité et donne également plusieurs pics parmi lesquels l'un correspond aux glycopeptides et aux glycoprotéines de l'invention possédant des propriétés anticoagulantes.

Selon un mode de réalisation préféré, le procédé d'obtention des glycopeptides et des glycoprotéines de l'invention comprend:

la séparation du surnageant provenant d'un milieu de culture de myxobactéries du sous-ordre des Cystobacterinae ou de mutants de celles-ci, laquelle culture est au début de la phase stationnaire de croissance;

l'addition d'alcool au susdit surnageant, ce qui conduit à l'obtention d'un précipité qui contient les glycopeptides et les glycoprotéines selon l'invention et qui est débarrassé des substances de poids moléculaires inférieurs à environ 1 000 daltons;

le lavage du susdit précipité à l'acétone, puis à l'éther, puis le séchage du précipité;

la dissolution dans l'eau distillée du susdit précipité séché;

l'élimination des substances insolubles dans l'eau, notamment par centrifugation et l'obtention d'une solution contènant les glycopeptides et les glycoprotéines de l'invention et débarrassée des substances de poids moléculaires inférieurs à environ 1 000 daltons, et des substances insolubles dans l'eau;

la purification par ultrafiltration et la concentration de la solution obtenue ci-dessus, ce qui conduit à l'obtention d'une fraction contenant les glycopeptides et les glycoprotéines;

la conservation de la susdite fraction par lyophilisation ou évaporation sous vide;

la purification par chromatographie sur colonne de pseudo-affinité de la susdite fraction;

la purification subséquente par chromatographie sur colonne de gel de polymère réticulé de dextrane, notamment de gel commercialisé sous le nom Sephadex.

Selon un autre mode de réalisation préféré du procédé selon l'invention, celui-ci comprend en plus des étapes ou phases décrites ci-dessus, une étape d'élimination des protéines. Cette étape a lieu avant l'étape de purification par chromatographie, à un moment quelconque entre la séparation du surnageant du milieu de culture des myxobactéries et la purification par chromatographie sur colonne de pseudo-affinité.

L'élimination des protéines peut avoir lieu par exemple en utilisant du phénol, mais peut également avoir lieu en utilisant une membrane qui permet d'éliminer les substances de poids moléculaires supérieurs à 10 000 daltons.

Si on utilise le phénol pour éliminer les protéines, il se produit un précipité qui contient les protéines à éliminer, tandis que les glycopeptides et les glycoprotéines selon l'invention se trouvent dans la phase aqueuse obtenue.

Le phénol utilisé est du phénol d'environ 40 à environ 70% (poids/volume) et est de préférence du phénol à environ 50% (poids/volume).

Selon un mode de réalisation préféré, le procédé de l'invention comprend:

la séparation et la récupération du surnageant provenant d'un milieu de culture de myxobactéries du sous-ordre des Cystobacterinae ou de mutants de celles-ci;

l'élimination à partir du surnageant ainsi récupéré;

des protéines,

des substances de poids moléculaires inférieurs à environ 1 000 daltons,

des substances insolubles dans l'eau; pour donner l'extrait brut,

la purification par chromatographie sur colonne de pseudo-affinité de l'extrait brut;

la purification subséquente par chromatographie sur colonne de gel commercialisé sous le nom Sephadex.

Un mode de réalisation préféré du procédé de l'invention, pour obtenir les glycopeptides et les glycoprotéines selon l'invention, comprend les étapes suivantes:

la séparation et la récupération du surnageant provenant d'un milieu de culture de myxobactéries appartenant au groupe produisant un mucus colorable au rouge Congo, laquelle culture est au début de la phase stationnaire de croissance;

l'addition au surnageant de phénol à 50% (poids/volume), qui conduit à:

l'obtention d'un précipité contenant les protéines;

et à l'obtention d'une phase aqueuse contenant les glycopeptides et les glycoprotéines selon l'invention;

la séparation des phases et la récupération de la phase aqueuse qui contient les glycopeptides et les glycoprotéines selon l'invention;

l'addition d'alcool à la susdite phase aqueuse qui conduit à l'obtention d'un précipité qui contient les glycopeptides et les glycoprotéines selon l'invention et qui est débarrassé des protéines et des substances de poids moléculaires inférieurs à environ 1 000 daltons;

le lavage du susdit précipité à l'acétone, puis à l'éther, puis le séchage du précipité;

la dissolution dans l'eau distillée du susdit précipité séché;

l'élimination des substances insolubles dans l'eau, notamment par centrifugation et l'obtention d'une solution contenant les glycopeptides et les glycoprotéines de l'invention et débarrassée des substances de poids moléculaires inférieurs à environ 1 000 daltons, des protéines et des substances insolubles dans l'eau;

la purification par ultrafiltration et la concentration de la solution obtenue ci-dessus, ce qui conduit à l'obtention d'un extrait brut contenant les glycopeptides et les glycoprotéines;

la conservation du susdit extrait brut par lyophilisation ou évaporation sous vide;

la purification par chromatographie sur colonne de pseudo-affinité de l'extrait brut;

la purification subséquente par chromatographie sur colonne de gel commercialisé sous le nom Sephadex.

De façon avantageuse, le traitement à l'alcool de la phase aqueuse contenant les glycopeptides et les glycoprotéines selon l'invention peut être effectué successivement deux fois.

De façon avantageuse, on peut laver avec de l'eau distillée les substances insolubles obtenues et procéder à une centrifugation.

Selon un mode de réalisation avantageux, le procédé d'obtention des glycopeptides et des glycoprotéines de l'invention comprend en outre une étape dans laquelle on traite le surnageant afin d'éliminer les protéases, notamment par chauffage à environ 100°C, pendant un temps suffisant, par exemple pendant environ 2 à environ 5 min.

On indique ci-après un procédé de dénaturation des protéases et d'extraction des glycopeptides de l'invention. Ce procédé comprend les étapes suivantes:

l'élimination des bactéries par centrifugation à 8 000 g pendant 15 minutes à partir d'une culture de préférence en début de phase stationnaire de croissance,

la mise en contact du surnageant de culture récupéré dans un bain d'eau bouillante à 100°C pendant 2 à 5 minutes tout en agitant en permanence,

le refroidissement du surnageant dans un bain d'eau glacée, avec agitation,

la lyophilisation du surnageant,

la dissolution de la poudre (obtenue par la lyophilisation ci-dessus) dans le PBS (Phosphate Buffered Saline) à pH 7,2, à raison d'un ml de PBS pour 10 ml de surnageant initial,

l'addition au concentrat obtenu de 3 volumes d'alcool 95° et la mise au repos pendant une nuit à −20°C,

la centrifugation à 8 000 g pendant 15 minutes et l'élimination de la phase aqueuse,

la resuspension du précipité dans l'eau distillée,

la reprécipitation avec 3 volumes d'alcool 95° à −20°C pendant au moins 4 heures,

le lavage du précipité à l'acétone et à l'éther,

le séchage sur une paillasse à flux laminaire à la température ambiante,

la resuspension dans l'eau distillée,

l'élimination des substances insolubles dans l'eau par centrifugation pendant 4—5 minutes à 160 g,

le lavage à 2 reprises des substances insolubles à l'eau distillée et la centrifugation comme précédemment,

la filtration de la solution sur une membrane Amicon 1000D, à la température ambiante,

la lyophilisation,

la mise en oeuvre des étapes de purification par chromatographie.


Exemple 1

L'exemple 1 ci-après est relatif à l'obtention d'un glycopeptide selon l'invention.

La souche de myxobactéries utilisée est la souche *Myxococcus xanthus* CM011.

Les cellules sont cultivées en milieu liquide (10 g/l de Bactocasitone commercialisé par Difco; 8 mM $MgSO_4$; Tris[(hydroxyméthyl)amino-méthane] pH 7,6 10 mm; tampon phosphate pH 7,6 1 mM) à 30°C sous forte aération (agitation rotative à 300 rpm en erlen ou fermenteur de 7 l).

On prend la culture de myxobactéries en début de phase stationnaire de croissance et on centrifuge à 8 000 g pendant environ 15 à 30 minutes pour éliminer les bactéries et récupérer le surnageant du milieu de culture.

On ajoute au surnageant un volume de phénol à 50% (poids/volume) dans l'eau distillée.

On agite énergiquement et on laisse agir à 60°C pendant 5 minutes.

On refroidit directement à 0°C dans un mélange réfrigérant (glace+eau) et on laisse reposer pour séparer la phase aqueuse du précipité qui s'est formée suite à l'addition de phénol.

On prélève la phase aqueuse qui contient le glycopeptide selon l'invention, on centrifuge à 1 500 g à 0°C pendant 2 à 3 minutes et on récupère la phase aqueuse.

On effectue un second traitement au phénol en ajoutant du phénol à 50% à la phase aqueuse qui vient d'être obtenue.

On traite ensuite la phase aqueuse avec 5 volumes d'alcool 95° à −20°C pendant une nuit, ce qui permet de précipiter le glycopeptide de l'invention et d'éliminer le phénol et de concentrer.

On centrifuge à 3 000 g et à 0°C pendant 15 à 30 minutes.

On resuspend le précipité dans un petit volume d'eau distillée et on reprécipite avec 3 volumes d'alcool 95° à −20°C (comme précédemment).

On lave le précipité obtenu à l'acétone, ce qui permet notamment de délipider partiellement, d'éliminer le phénol restant éventuellement, puis à l'éther (à −20°C), ce qui permet d'éliminer le phénol restant éventuellement et on laisse sécher par exemple sur une paillasse à flux laminaire.

On redissout le précipité dans l'eau distillée.

On élimine les substances insolubles dans l'eau par centrifugation pendant 4—5 minutes à 160 g.

On lave les substances insolubles avec de l'eau distillée et on centrifuge comme précédemment.

On ultrafiltre pour éliminer notamment l'alcool, l'acétone et l'éther et on concentre par exemple en utilisant une membrane Amicon® 1000D.

On obtient ainsi ce que l'on a précédemment appelé l'extrait brut (et qui sera ci-après appelé extrait phénolique brut en raison de l'utilisation du phénol indiquée ci-dessus) qui contient le glycopeptide selon l'invention.

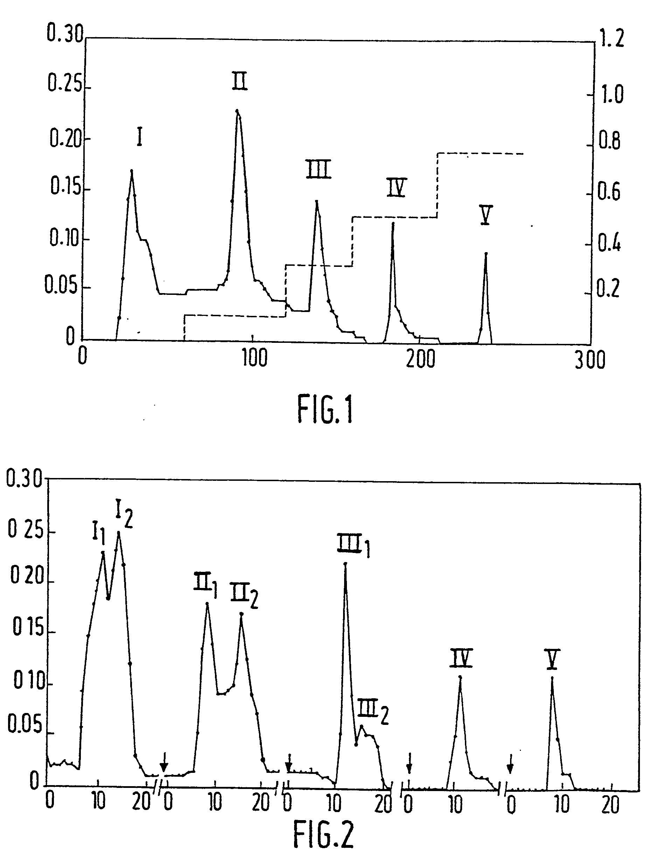

La figur 1 représente la courbe 1 relative à la chromatographie de pseudo-affinité sur histidyl-(sépharose-4B) de l'extrait brut.

On a représenté sur l'axe des abscisses, les volumes d'élution en ml et sur l'axe des ordonnées, à droite, la concentration molaire en NaCl et sur l'axe des ordonnées à gauche, la densité optique mesurée à 280 nm.

La colonne utilisée a pour dimensions 1×25 cm.

L'échantillon est constitué par 25 mg d'extrait phénolique brut lyophilisé dans 1 ml de tampon.

L'éluant est constitué par du tampon acétate 0,1 M. Le pH est de 5,5 et le gradient de NaCl est un gradient par palier de 0 à 0,75 M. Le débit est de 12 ml à l'heure.

Cette séparation conduit à l'obtention de cinq fractions correspondant aux cinq pics respectivement désignés par I, II, III, IV et V sur la figure 1.

Le pic II obtenu avec NaCl 0,1 M dans le tampon correspond à une fraction présentant des propriétés anticoagulantes et contient le glycopeptide selon l'invention.

La figure 2 représente la courbe 2 relative à la filtration sur gel commercialisé sous le nom Séphadex G-25 des différentes fractions obtenues par histidylsépharose.

On a représenté sur l'axe des abscisses le volume d'élution en ml, sur l'axe des ordonnées, la densité optique mesurée à 280 nm.

La colonne a une dimension de 1×22 cm.

L'éluant est constitué par de l'eau distillée et le débit est de 16 ml/heure.

Le glycopeptide selon l'invention correspond au pic représenté par II(2) sur la figure 2.

La flèche ( ↓ ) indique le début de chaque élution.

La pureté du glycopeptide selon l'invention est vérifiée par électrofocalisation (cf. Anders Winter, Krishna Ek. Ulla-Birgitta Anderson, Analytical Electrofocusing in thin layers of polyacrylamide gels, LKB Application Note 250, 1977).

Le glycopeptide selon l'invention représente environ 2% du susdit extrait phénolique brut.

Exemple 2

Cet exemple est relatif à l'obtention du glycopeptide obtenu à partir de l'extrait phénolique brut mentionné ci-dessus.

On a isolé le glycopeptide selon l'invention à partir de l'extrait phénolique brut obtenu notamment comme indiqué ci-dessus, à l'aide d'une colonne de silice connue sous la désignation (C8-RP300), en fonction de l'hydrophobicité.

En présence ou absence de SDS (sodium dodécyl sulfate), il présente essentiellement deux pics contenant à la fois des résidus sucres et peptides. Le glycopeptide selon l'invention est contenu dans la fraction correspondant au pic le plus hydrophile (65% en masse), la fraction correspondant à l'autre pic contient un principe coagulant.

Le sodium dodécyl sulfate élimine l'activité anticoagulante du premier pic, mais n'en modifie pas la migration.

Exemple 3

Cet exemple est relatif à l'obtention des glycopeptides selon l'invention en ayant recours au procédé indiqué ci-dessus dans lequel on effectue uniquement la précipitation à l'alcool.

Ce procédé ne diffère de celui déjà décrit à l'exemple 1 que par l'élimination de l'étape correspondant à la précipitation à l'aide du phénol. La séparation du glycopeptide est rendue possible par une chromatographie de pseudo-affinité sur un gel d'histidyl-sépharose suivie par une filtration sur gel G-25 dans les conditions déjà décrites à l'exemple 1. L'homogénéité du produit a été vérifiée par électrofocalisation et son pH isoélectrique déterminé (environ 3,6).

On indique ci-après, en détail, les protocoles d'extraction et de purification.

On prend la culture de *Myxococcus xanthus* CM011 au début de la phase stationnaire de croissance.

On élimine les cellules par centrifugation à 8 000 g pendant 15 à 30 minutes et on récupère le surnageant.

On traite le surnageant avec 3 volumes d'alcool à 95° et on laisse pendant 4 heures ou une nuit à −20°C.

Pour séparer le précipité formé de la phase aqueuse, on centrifuge la phase aqueuse à 8 000 g pendant 15 à 30 minutes et on élimine la phase aqueuse.

On resuspend le précipité dans de l'eau distillée.

On reprécipite la phase aqueuse avec 3 volumes d'alcool à 95° à −20°C pendant au moins 4 heures.

On lave le précipité obtenu à l'acétone puis à l'éther.

On laisse sécher le précipité sur une paillasse à flux laminaire à la température ambiante.

On suspend le précipité dans de l'eau distillée.

On élimine les substances insolubles dans l'eau par centrifugation pendant 4—5 minutes à 160 g.

On lave les substances insolubles avec de l'eau distillée et on centrifuge comme précédemment.

On filtre la solution sur une membrane Amicon® 1000D, à la température ambiante.

On lyophilise, ce qui conduit à l'obtention d'un lyophilisat.

On procède ensuite à une chromatographie de pseudo-affinité du lyophilisat sur histidyl-(sépharose 4B).

On procède ensuite à une filtration sur une colonne de gel commercialisée sous le nom Sephadex®G-25 des différentes fractions obtenues sur histidyl-Sépharose®-4B).

Le gel utilisé est la Sépharose®-4B, commercialisée par Pharmacia Fine Chemicals, couplée à un ligand L-histidine, commercialisé par la Société Sigma.

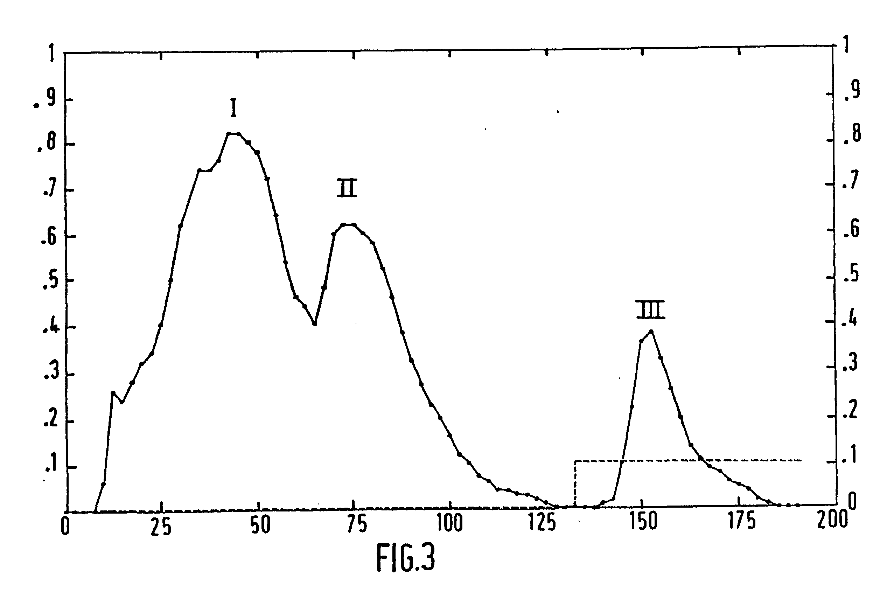

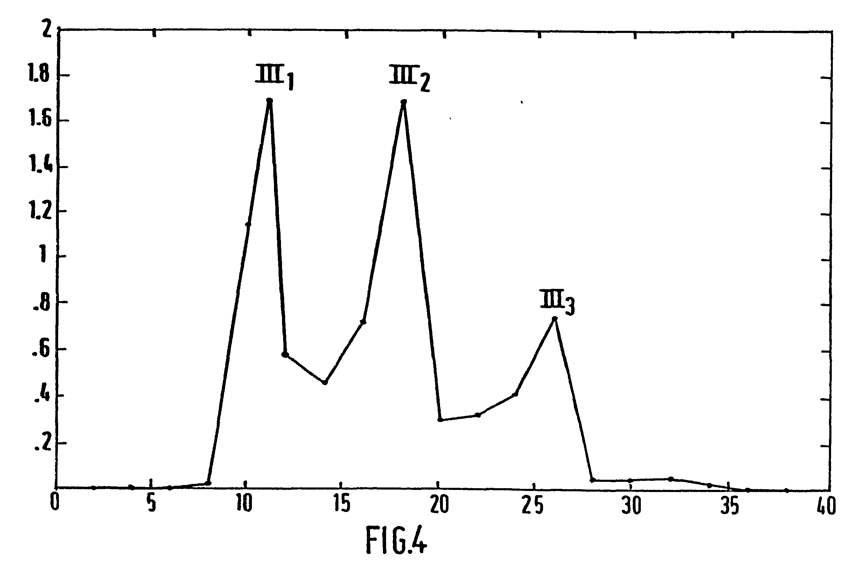

On a représenté respectivement sur les figures 3 et 4, les courbes relatives à la chromatographie de pseudo-affinité et de filtration sur gel G-25, pour l'obtention du glycopeptide selon l'invention.

La figure 3 représente les trois pics (I, II, III) obtenus par la séparation sur histidyl-(sépharose 4B).

On a représenté sur l'axe des abscisses le volume d'élution exprimé en ml, sur l'axe des ordonnées, à droite, la concentration en NaCl et à gauche la densité optique à 280 nm.

La colonne utilisée a comme dimensions 2,5×16 cm.

L'échantillon est constitué par 1 g de lyophilisat dans 10 ml de tampon.

L'éluant est un tampon acétate 0,1 M, pH 5,5, le débit est de 48 ml/heure.

L'élution est faite avec un gradient de NaCl (0—0,75 M) dans le tampon.

C'est dans la fraction correspondant au pic III que se trouve le glycopeptide présentant des propriétés anticoagulantes selon l'invention, éluée avec NaCl, 0,1 M dans le tampon.

10

La fraction correspondant au pic III obtenu sur séparation histidyl-(sépharose 4B) est concentrée par ultrafiltration sur membrane Amicon® 1000D et est filtrée sur gel G25.

La figure 4 représente la filtration sur gel G-25 du pic III obtenue précédemment.

On a représenté en abscisses le volume d'élution exprimé en ml, et en ordonnées, la densité optique à 280 nm.

La colonne utilisée est une colonne de 1×25 cm.

L'éluant est de l'eau distillée.

Le débit est de 16 ml/heure.

Le glycopeptide présentant des propriétés anticoagulantes selon l'invention, correspond au pic $III_1$.

Analyse du glycopeptide

La fraction peptidique a été mise en évidence après dosage des protéines totales selon la méthode de Lowry.

Selon cette methode, le glycopeptide contient 79—80% (en poids) de résidus acides aminés.

La partie glucidique du glycopeptide de l'invention correspond à 20% environ (en poids) de glucides totaux.

La fraction glucidique a été analysée par chromatographie en phase gazeuse après méthanolyse.

La technique de méthanolyse est la suivante.

Pour déterminer la composition de 20 µg de sucres:

on met 2 µg de mésoinositol (témoin interne) à 1 µg/ml dans de l'eau distillée;

on lyophilise;

on met 500 µl de méthanol anhydre 0,5 N/HCl et on laisse à 95°C pendant 24 heures;

on sort les tubes du four pour arrêter la méthanolyse, on laisse refroidir à la température ambiante et on neutralise jusqu'à ce que le pH final soit de 6—7;

on met 20 µl d'anhydride acétique dans le mélange, on agite et on laisse réagir pendant une nuit;

on centrifuge à 250 g, on élimine l'insoluble et on ajoute sur le surnageant de l'heptane (250 µl), on agite et on enlève l'heptane qui est non miscible avec le méthanol, afin d'extraire les traces de lipides;

on répète cette extraction plusieurs fois;

on sèche à l'azote;

on silyle pour stabiliser les produits et les rendre plus volatils;

on met 100 µl de pyridine et 100 µl de BSTFA (N,O-bis-(triméthylsilyl)-trifluoroacétamide), on laisse à l'obscurité pendant 4 heures;

on injecte sur une colonne de OV101 Cp Sil 5 CB Chrompach, la température est programmée de 120 à 240°C, à raison de 2°C/mn, sous un débit de gaz vecteur (azote) de 10 ml/mn.

La composition centésimale (en poids) de cette fraction en résidus sucres est donnée dans le tableau suivant.

| Glc | Man | Gal | Glc Nac | Gal Nac |
|------|------|------|---------|---------|
| 34,9% | 16,3% | 11,4% | 23,3% | 14,1% |
| 62,6% de sucres neutres | | | 37,4% d'osanines | |

Glc=glucose
Man=mannose
Gal=galactose
Glc Nac=N-acétylglucosamine
Gal Nac=N-acétylgalactosamine.

L'analyse des acides aminés est effectuée à l'aide d'un autoanalyseur Beckman multichrom selon la technique de Spakman et Coll. (Spakman D. H., Stein W. A. and Tioore S. 1958 automatic recording apparatus for use in the chromatography of amino-acids. Analyt. Chem. 30:1190).

La composition centésimale (en poids) de cette fraction en résidus d'acides aminés figure ci-après:

Acides aminés

| | |
|---|---|
| Glu | 31% |
| Ser | 28% |
| Asp | 7% |
| Gly | 6% |
| Ile | 5,5% |
| Ala | 5% |
| Val | 3% |
| Leu | 3% |
| Thr | 2,5% |
| Tyr | 2,5% |
| Pro | <2% |
| Lys | <2% |
| Phe | <2% |
| His | <2% |
| Arg | <2% |

D'autres acides non mentionnés ici ont été détectés à l'état de traces.

Détermination du poids moléculaire et du caractère glycopeptidiques du glycopeptide de l'invention

Le poids moléculaire estimé par un procédé d'électrophorèse sur gel de polyacrylamide (urée-SDS-Page) est d'environ 6 300 D. (figure 1C).

Le procédé "Urée-SDS-Page" est réalisé selon un protocole fourni par Pharmacia sur un gel de 13,4% d'acrylamide et de 1 mm d'épaisseur. L'urée est introduite à une concentration de 8 M dans le gel de résolution et de 4 M dans le gel de concentration.

Les tampons sont du tris-glycine 2 M, pH 8,8 pour le gel de résolution et du tris-glycine 0,5 M, pH 6,8 pour le gel de concentration. Les échantillons sont solubilisés dans un tampon tris 0,5 M, pH 6,8 contenant du sodium dodécylsulfate (SDS) (4%),

du mercaptoéthanol (1%),

de l'urée 7 M,

du saccharose (12%),

puis sont portés à 100°C pendant 5 mn.

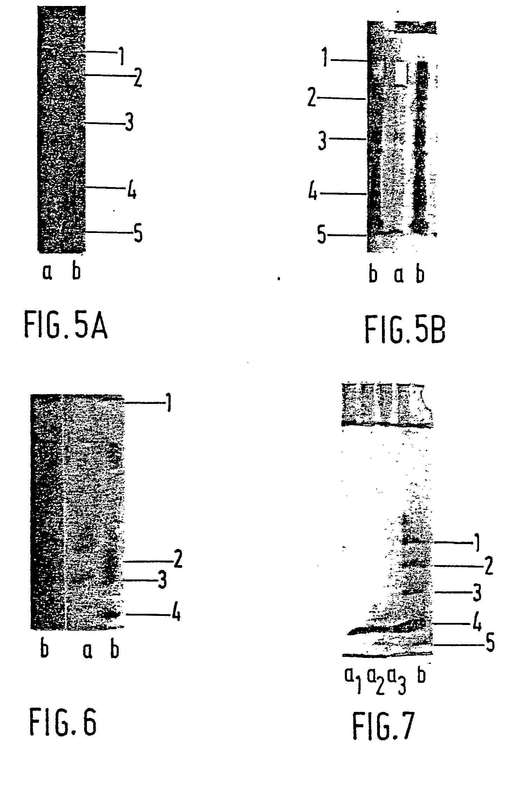

Sur la figure 7, on a représenté en

1 la bande correspondant à la myoglobine (17200 D)

2 la bande correspondant à la myoglobine I et II (14600 D)

3 la bande correspondant à la myoglobine I (8240 D)

4 la bande correspondant à la myoglobine II (6380 D)

5 la bande correspondant à la myoglobine III (2560 D).

Sur la figure 7,

la bande a1 correspond à une concentration 0,8 mg/ml du glycopeptide de l'invention,

la bande a2 correspond à une concentration 1 mg/ml du glycopeptide de l'invention,

la bande a3 correspond à une concentration 2 mg/ml du glycopeptide de l'invention,

la bande b correspond au kit de calibration.

La détermination de l'homogénéité du produit et du caractère glycopeptidique du glycopeptide a été réalisée par une électrofocalisation sur gel d'acrylamide à 5% (Pharmacia) suivie d'une coloration à l'argent selon deux techniques différentes. La première est celle commercialisée par Biorad, pour la coloration des peptides (Merril C. R., Goldman D., Desman S. A., and Ebert M. H. 1981 "Ultrasensitive stain for proteins in polyacrylamides gels shows regional variation in cerebrospiral fluid proteins, Science 211: 1437—1438).

La deuxième comprenant une étape d'oxydation périodique, permet de révéler spécifiquement les sucres (Dubray G and Bezard G. 1982, "A highly sensitive periodic acid-silver stain for 1,2-Diol groups of glycopeptids and polysaccharids in polyacrylamide gels. Anal. Biochem. 119:325—329).

Dans les deux cas, la même bande a été révélée dans le gel ce qui constitue une preuve en faveur de la nature glycopeptidique de la substance (figures 5A et 5B).

Plus précisément, on a représenté en 5A l'électrofocalisation suivie de la coloration des peptides selon la première méthode indiquée ci-dessus.

On a représenté en 5B l'électrofocalisation suivie de la coloration à l'argent et comprend une étape d'oxydation périodique pour révéler les sucres, selon la deuxième méthode indiquée ci-dessus.

La bande a correspond au glycopeptide de l'invention tandis que la bande b correspond à la bande de contrôle du kit de calibration.

Sur les figures 5A et 5B, on a représenté par

1 la bande correspondant à la lectine de lentille—bande intermédiaire (pI=8.45)

2 la bande correspondant à la bande de base de la myoglobine (pI=7.35)

3 la bande correspondant à l'anhydrase carbonique humaine B (pI=6.55)

4 la bande correspondant à la β-lactoglobuline A (pI=5.2)

la bande correspondant à l'amyloglucosidase (pI=3.5).

On a représenté, sur la figure B, la détermination plus fine du point isoélectrique.

Sur la figure 6,

1 correspond à la bande de l'anhydrase carbonique bovine (pI=5.85)

2 correspond à la bande de la glucose oxydase (pI=4.15)

3 correspond à la bande de l'amyloglucosidase (pI=3.50)

4 correspond à la bande du pepsinogène (pI=2.80).

Propriétés des glycopeptides selon l'invention

L'effet des glycopeptides sur la coagulation a été investigué et leurs propriétés anticoagulantes ont été déterminées par un ensemble de tests de coagulation.

L'héparine qui a été utilisée à titre de comparaison est de l'héparine commercialisée par Choay S. A., de 10 300 daltons et de 173USP.

On rappelle qu'une unité internationale (UI) est définie comme étant la quantité de substance (en l'occurence de glycopeptide) qui attaque une micromole de substrat par minute et qu'une unité catalytique (Ukat) est la quantité de substance (en l'occurence de glycopeptide) qui attaque une mole de substrat par seconde.

Le glycopeptide selon l'invention à propos duquel les résultats figurent ci-après est celui obtenu dans l'exemple 3.

L'activité anticoagulante, déterminée par le temps de Howell est exprimé en unité internationale par mg de poids sec. Une unité internationale est définie comme étant la quantité de substance ayant le même effet sur le temps de coagulation qu'une unité internationale d'héparine.

Il faut ajouter qu'aucun signe de cytotoxicité n'a été constaté par des tests préliminaires effectués *in vitro* sur des fibroblastes de poumon de poulet.

Ces différents résultats proviennent des essais tels que décrits ci-dessous.

Exploration de la voie endogène de la coagulation:

a—APTT ou temps de céphaline kaolin

C'est le plus sensible des tests de la voie endogène. Il correspond au temps de coagulation d'un plasma qui est récalcifié en présence de céphaline et d'un activateur des facteurs de contact. L'activateur a longtemps été du kaolin, surface électronégative qui active fortement le système contact dont le premier évènement est l'activation et l'acquisition d'une activité enzymatique par le facteur XII (Facteur Hageman).

Ce temps explore tous les facteurs de la voie endogène.

Protocole expérimental: à 0,1 ml de PPP (plasma pauvre en plaquettes) préincubé à 37°C, on ajoute:

0,1 ml de tampon de Michaelis, pH 7.3 ou d'anticoagulant dans le tampon à différentes concentrations, on incube pendant 3 minutes à 37°C,

on déclenche la coagulation par l'addition de 0.1 ml d'activeur (céphaline+kaolin, commercialisé sous le nom C. K., Prest 2, par la Société Diagnostica stago I) et 0.1 ml de CaCl$_2$ M/40,

on mesure le temps de coagulation à l'aide d'un coagulomètre (KCl Dade).

b—Temps de Howell

Il correspond au temps de coagulation d'un plasma pauvre en plaquettes recalcifié. C'est un test voisin dans son principe du temps de coagulation sur sang total, sauf que la plasma est pauvre en plaquettes; il est plus court et ne dure environ que 2 à 3 minutes. Cette différence est liée à l'activation in vitro des facteurs de contact. Cette activation est indépendante et peut donc survenir dans le tube du prélèvement, malgré le citrate qui décalcifie le sang. Lorsque l'on restitue le calcium au plasma au moment de la réalisation du test, la cascade enzymatique démarre au niveau du facteur IX.

Protocole expérimental

A 37°C

on incube pendant 3 minutes 0,1 ml de PPP avec 0.1 ml de tampon ou de produit à tester dans le tampon à concentrations variables,

on ajoute 0.1 ml de CaCl$_2$ M/40 pour déclencher le phénomène de coagulation,

on chronométre.

II. Exploration de la voie exogene de la coagulation

Un seul test explore la voie exogène, le temps de Quick. Il correspond au temps de coagulation d'un plasma que l'on recalcifie en présence de thromboplastine tissulaire, source de facteur III. La néoplastine (Diagnostica Stago) préparée à partir de tissu cérébral frais, est la seule thromboplastine qui permet d'explorer sélectivement, en toutes circonstances, la prothrombine et ses cofacteurs constituant ce qu'on appelle le complexe prothrombinique (II, V, VII, X).

Protocole

A 0,1 ml de plasma préincubé à 37°C, on ajoute 0,1 ml de tampon ou d'anticoagulant à différentes concentrations dans le tampon,

on laisse agir pendant 5 mn à 37°C,

on introduit dans le mélange réactionnel 0,2 ml de néoplastine portée au préalable à 37°C dans un bain-marie,

on mesure au coagulomètre le temps que met le caillot plasmatique à se former.

### III. Temps de thrombine

Le temps de thrombine (TT) correspond au temps de formation d'un caillot après addition de thrombine dans un échantillon de plasma ou une solution de fibrinogène, en présence ou en l'absence de glycopeptide de l'invention.

L'héparine Lot H 108 commercialisé par "Choay" est testée dans les mêmes conditions afin de servir d'élément de comparaison.

Le système expérimental est le suivant:

0,2 ml de plasma pauvre en plaquettes (PPP) ou de fibrinogène (4 g/l) est incubé à 37°C avec:

0,1 ml de tampon de Michaelis contenant le glycopeptide de l'invention ou l'héparine en concentration initiale C variable, le temps témoin étant déterminé avec un même volume de tampon exempt de produit, la durée d'incubation étant de 5 mn,

0,1 ml de solution de thrombine commercialisé par "Roche" et présentant les caractéristiques suivantes 56,4 u.NIH/mg (pour usage in vitro), gardée à 4°C dans du tampon de Michaelis est ajouté et le temps d'apparition du caillot (TT) est mesuré.

Les mesures sont faites à l'aide du coagulomètre.

### IV. Evaluation de l'activite Anti Xa

Elle consiste à mesurer l'effet potentialisateur du glycopeptide de l'invention, ayant l'héparine comme témoin, sur le pouvoir anti X plasmatique par méthode chronométrique.

Le complexe héparine-antithrombine III exerce une action inhibitrice sur les sérines protéases de l'hémostase. L'action inhibitrice sur le facteur Xa est largement responsable de l'action anticoagulante de l'héparine particulièrement aux faibles doses.

Le dosage se décompose en 3 temps:

1. Formation du complexe AT III-héparine par addition d'AT III purifiée à l'héparine selon le schéma suivant

$$\text{ATIII} + \text{héparine} \rightarrow \text{AT III} - \text{héparine}$$

2. Action inhibitrice du complexe formé, sur un excès connu de facteur Xa purifié suivant le schéma suivant

$$\text{ATIII} - \text{Hép.} + \text{Xa en excès} \rightarrow \text{ATIII} - \text{Hép.} - (\text{Xa}) + \text{Xa résiduel}$$

3. Mesure de l'activité enzymatique du facteur Xa résiduel par son action coagulante sur un plasma substrat suivant le schéma suivant

$$\text{Xa résiduel} + \text{plasma substrat} \rightarrow \text{caillot de fibrine.}$$

Activité anti-Xa

\* les réactifs sont les suivants: Facteur Xa purifié reconstitué par 1 ml d'eau distillée et gardé à 4°C,

HEPACLOT 10 STAGO: antithrombine III purifiée reconstituée par 0,5 ml d'eau distillée et placée à 4°C plasma substrat spécialement traité redissous dans 1 ml d'eau distillée et incubé à 37°C,

\* L'étalonnage est effectué avec la calciparine CHOAY (173 UI/mg dans une gamme de 0—0,5 UI/ml).

\* Mode opératoire: on considère que cette héparine titre par définition 173 Unités anti Xa/mg. Dans un tube plastique à 37°C, on met

50 µl de tampon de Michaelis, de l'héparine ou du glycopeptide de l'invention dans le même volume du tampon à concentrations initiales variables,

50 µl d'antithrombine III, et on incube pendant 60 à 37°C,

on ajoute: 100 µl de facteur Xa et on incube exactement pendant 90 s,

100 µl de plasma substrat (à 37°C) et on incube pendant 30 s,

on déclenche le phénoméne de coagulation par l'addition de 100 µl de $CaCl_2M/40$ préincubé à 37°C, on note le temps de coagulation.

### V. Temps de reptilase (TR)

C'est le temps de coagulation du plasma citraté en présence de reptilase R (Diagnostica Stago). Le réactif est constitué par la fraction thrombine extraite du venin du serpent Biotrops Atrox. Contrairement à la thrombine, la reptilase n'est pas sensible à l'héparine et n'est pas inhibée par l'AT III. Elle est capable de provoquer la polymérisation du fibrinogène. Le temps de reptilase permet donc d'évaluer la capacité d'une substance de modifier ou non la transformation du fibrinogène en fibrine.

Description du test

Ce test consiste à incuber à 37°C 0,2 ml de fibrinogène 4 mg/ml (CRTS—Lille) avec 0,1 ml de tampon. Le glycopeptide de l'invention ou l'héparine sont dissous dans un même volume de tampon à différentes concentrations et ajoutés à 0,2 ml de fibrinogène 4 mg/ml.

Après incubation de 3 mn, on ajoute 0,1 ml de reptilase (lyophilisée, remise en solution dans l'eau distillée et gardée à 4°C) au mélange réactionnel pour déclencher le phénomène de coagulation et le temps de formation du caillot de fibrine est mesuré.

Resultats

1. APTT ou temps de céphaline kaolin

L'héparine "CHOAY Lot H-108, 10700D" est choisie comme référence et son effet au niveau de cette voie est aussi bien évalué que celle du glycopeptide.

On a rassemblé dans le tableau 1, ci-après, les valeurs de l'APTT à différentes concentrations d'héparine.

TABLEAU 1

| Heparine (µg/ml) | 0 | 0,15 | 0,23 | 0,58 | 0,87 | 2,89 | 5,78 | 6,35 |
|---|---|---|---|---|---|---|---|---|
| Moyenne des temps de coagulation (secondes) | 67,5 | 69,9 | 80 | 95,3 | 110 | 237,5 | 440 | ∞ |

On a rassemblé dans le tableau 2, ci-après, les valeurs l'APTT à différentes concentrations de glycopeptide de l'invention.

TABLEAU 2

| Glycopeptide de l'invention (µg/ml) | 0 | 10 | 20 | 40 | 50 | 75 | 90 | 100 |
|---|---|---|---|---|---|---|---|---|
| Moyenne des temps de coagulation (secondes) | 67,5 | 64 | 62,9 | 85 | 93,5 | 136,2 | 201 | ∞ |

D'après ces résultats, on décèle une propriété procoagulante manifestée par le glycopeptide de l'invention à des concentrations inférieures à 30 µg/ml. Au delà de cette concentration, le glycopeptide de l'invention allonge le temps de céphaline kaolin jusqu'à empêcher complètement la formation du caillot à partir de 100 µg/ml. L'héparine quant à elle n'a pas cet effet procoagulant et à 6,35 µg/ml inhibe totalement la coagulation.

Cette comparaison nous permet de définir l'activité du glycopeptide de l'invention par rapport à celle de l'héparine.

En effet, le glycopeptide de l'invention évalué par l'APTT est environ 42 fois moins actif que l'héparine. La concentration aboutissant à l'allongement de 2 fois de l'APTT était de 70 µg/ml pour le glycopeptide de l'invention et 1,06 µg/ml pour l'héparine.

2. Temps de QUICK

On évalue par ce test l'action du glycopeptide de l'invention au niveau du complexe prothrombinique (II, V, VII, X), prenant l'héparine CHOAY comme référence, les résultats figurent dans les tableaux 3 et 4 pour l'héparine et le glycopeptide de l'invention respectivement.

TABLEAU 3
Temps de quick à différentes concentrations d'héparine

| Heparine (µg/ml) | 0 | 2,89 | 5,78 | 14,45 | 28,90 | 43,35 | 57,80 | 75,25 | 86,70 |
|---|---|---|---|---|---|---|---|---|---|
| Moyenne des temps de coagulation (secondes) | 16,7 | 16,5 | 16,6 | 17 | 17,7 | 21,4 | 35,8 | 118,9 | ∞ |

TABLEAU 4

Temps de quick à différentes concentrations de glycopeptide de l'invention

| Glycopeptide de l'invention (µg/ml) | 0 | 25 | 50 | 80 | 120 | 160 | 200 | 240 |
|---|---|---|---|---|---|---|---|---|
| Moyenne des temps de coagulation (secondes) | 16,7 | 16,5 | 16,8 | 19,5 | 21 | 35,5 | 116 | ∞ |

La concentration en glycopeptide de l'invention nécessaire pour doubler le temps de QUICK est de 152 µg/ml environ, alors que celle de l'héparine capable de produire le même effet est de 56 µg/ml, ce qui amène à conclure que sur la voie exogène de la coagulation, l'activité de l'héparine est 2,7 fois celle du glycopeptide de l'invention.

3. Temps de thrombine (TT) sur plasma

Ce test permet d'évaluer l'activité antithrombique de l'ancticoagulant dans un milieu plasmatique, hétérogène et en présence de l'antithrombique III présente normalement dans le plasma. Les résultats relatifs à l'héparine et à le glycopeptide de l'invention sont présentés respectivement dans les tableaux 5 et 6, ci-après, la thrombine étant utilisée à 20 uNIH/ml (uNIH—National Institute Health—: quantité de fraction coagulante de thrombine nécessaire pour coaguler en 15 s une solution de fibrinogène à 2°/00 tamponnée à 7,4, sous un volume final de 1 ml à 37°C).

TABLEAU 5

TT et concentration en thrombine inactivée en fonction de la concentration en héparine (µg/ml)

| Heparine (µg/ml) | 0 | 0,23 | 0,58 | 1,16 | 2,9 | 5,78 | 11,56 | 15 | 30 |
|---|---|---|---|---|---|---|---|---|---|
| TT (en secondes) | 5,3 | 6 | 6,5 | 7 | 10 | 35 | 94,5 | ∞ | ∞ |
| T. inactivée uNIH/ml | — | 2,143 | 3,34 | 5,3 | 10 | 16,25 | 19 | 20 | 20 |

TABLEAU 6

TT et concentration en thrombine inactivée en fonction de la concentration en glycopeptide de l'invention (µg/ml)

| Glycopeptide de l'invention (µg/ml) | 0 | 5 | 10 | 20 | 30 | 50 | 70 | 90 | 100 | 110 |
|---|---|---|---|---|---|---|---|---|---|---|
| TT (en secondes) | 5,3 | 5,25 | 5,8 | 6,5 | 8,5 | 13 | 28 | 89,3 | ∞ | ∞ |
| T. inactivée uNIH/ml | — | — | 1,49 | 3,34 | 8,1 | 12,31 | 17 | 19 | 20 | 20 |

Etude du temps de thrombine (TT) à différentes concentrations de thrombine.

Le temps de coagulation à différentes concentrations de thrombine permet en traçant la courbe de TT (Temps de thrombine en seconde) en fonctions de 1/T (l'inverse de la concentration en thrombine (uNIH/ml)$^{-1}$ de déterminer la concentration en thrombine inactivée connaissant la concentration en thrombine initiale pour chaque temps de coagulation.

Le tableau 7, ci-après, correspond au temps de thrombine (TT) mesuré sur plasma à différentes concentrations de thrombine.

EP 0 219 400 B1

TABLEAU 7

TT mesurée sur plasma à différentes concentrations de thrombine

| Concentration en T. (uNIH/ml) | 5 | 10 | 15 | 20 | 30 |
|---|---|---|---|---|---|
| I/T (uNIH)$^{-1}$ | 0,2 | 0,1 | 0,066 | 0,05 | 0,033 |
| T.T. (en secondes) | 19,5 | 10 | 7,5 | 5,3 | 4,5 |

Le tableau 8, ci-après, représente le temps de coagulation mesuré sur fibrinogène à différentes concentrations de thrombine.

TABLEAU 8

| Concentration en T. (uNIH/ml) | 5 | 7,5 | 10 | 15 | 20 |
|---|---|---|---|---|---|
| 1/T (uNIH/ml)$^{-1}$ | 0,2 | 0,133 | 0,1 | 0,066 | 0,05 |
| Temps de coagulation (secondes) | 49,2 | 31 | 23,5 | 15,9 | 12,5 |

En prenant comme critère la concentration d'héparine ou de glycopeptide de l'invention nécessaire à l'inactivation de la moitié de la quantité de thrombine ajoutée au milieu réactionnel, on constate que:

1 mg d'héparine inactive 3 125 uNIH de thrombine environ, 1 mg de glycopeptide de l'invention inactif 278 uNIH environ, ce qui permet de constater que l'activité anti IIa du glycopeptide de l'invention dans le plasma est donc de 15,4 UI/mg environ ce qui corresond à environ 9% de l'activité héparinique à ce niveau.

3-1. Temps de thrombine (TT) sur fibrinogène

Un allongement du temps de thrombine sur fibrinogène s'explique soit par une altération du fibrinogène soit par une inactivation de la thrombine indépendante de l'antithrombine III.

Résultats:

La thrombine est utilisée à 20 uNIH/ml et l'héparine CHOAY sert de référence.

a) Inactivation de la thrombine par l'héparine. Les résultats sont rassemblés au tableau 9 ci-après.

TABLEAU 9

Activité antithrombique de l'héparine mesurée sur fibrinogène dans un milieu dépourvu d'antithrombine III

| Heparine (µg/ml) | 0 | 0,23 | 0,58 | 0,87 | 1,16 | 2,9 | 5,78 | 11,56 | 28,9 | 57,8 |
|---|---|---|---|---|---|---|---|---|---|---|
| T.T. (secondes) | 12,5 | 13 | 13,7 | 14,5 | 15,2 | 16,6 | 22,9 | 39 | 121,8 | ∞ |
| Thrombine inactivée (uNIH/ml) | 0 | 0,77 | 1,82 | 3,05 | 3,88 | 5,3 | 9,37 | 13,75 | 18 | 20 |

b) Inactivation de la thrombine par le glycopeptide de l'invention. Les résultats sont rassemblés au tableau 10 ci-après.

TABLEAU 10

Activité antithrombique du glycopeptide de l'invention évaluée sur fibrinogène dans un milieu dépourvu d'antithrombique III

| Glycopeptide (µg/ml) de l'invention | 0 | 5 | 10 | 20 | 25 | 50 | 70 |
|---|---|---|---|---|---|---|---|
| T.T. (en secondes) | 11,9 | 13 | 16 | 23,6 | 30 | 101 | ∞ |
| Thrombine inactivée (uNIH/ml) | 0 | 2,76 | 5,72 | 10 | 12,07 | 17,6 | 20 |

Comme précédemment, le critère d'évaluation quantitative de l'activité anti IIa est la quantité d'anticoagulant nécessaire à l'inhibition de 50% de la thrombine ajoutée au milieu réactionnel.
Ainsi:
1 mg d'héparine inhibe 1 538 uNIH de thrombine,
1 mg de glycopeptide de l'invention inhibe 500 uNIH de thrombine, ce qui correspond à environ 32% de l'activité héparinique constatée.

Activite Anti Xa

a) Inactivation du Xa par l'héparine. Les résultats sont rassemblés dans le tableau 11 ci-après.

TABLEAU 11

Temps de (Xa) à des concentrations variables en héparine

| Heparine (µg/ml) | 0 | 0,115 | 0,230 | 0,578 | 1,156 | 2,89 |
|---|---|---|---|---|---|---|
| Temps de coagulation (secondes) | 11 | 12,2 | 13,7 | 21,7 | 36,9 | 82,5 |
| $\log_t$ | 1,041 | 1,086 | 1,137 | 1,336 | 1,567 | 1,916 |

b) Inactivation du Xa par le glycopeptide de l'invention. Les résultats sont rassemblés dans le tableau 12 ci-après.

TABLEAU 12

Temps de Xa à des concentrations variables en glycopeptide de l'invention

| Glycopeptide de l'invention (µg/ml) | 0 | 2 | 4 | 8 | 12 | 16 | 20 |
|---|---|---|---|---|---|---|---|
| Temps de coagulation (secondes) | 11 | 11 | 12 | 16,1 | 19,8 | 29 | 37,2 |
| $\log_t$ | 1,041 | 1,041 | 1,079 | 1,21 | 1,3 | 1,463 | 1,57 |

Les courbes correspondantes représentent le logarithme du temps de coagulation ($\log_t$) en fonction de la concentration en glycopeptide de l'invention ou en héparine. Pour un même temps de coagulation (22s) choisi comme étant le double du temps témoin, les concentrations respectives d'héparine et de glycopeptide de l'invention nécessaire pour avoir le même effet anticoagulant sont respectivement 0,833 µg/ml et 11,83 µg/ml.

L'héparine CHOAY possède 173 UI/mg, par conséquent, et se rapportant à cette activité référence, le glycopeptide de l'invention évaluée pour son activité anti Xa possède: 12 UI/mg. Donc le glycopeptide de l'invention possède 7% de l'activité héparinique au niveau du facteur Xa.

Le temps de reptilase concernant respectivement l'héparine et le glycopeptide de l'invention est indiqué dans les tableaux 13 et 14 ci-après:

TABLEAU 13

| Hep. (UI/ml) | 0 | 2 | 5 | 10 |
|---|---|---|---|---|
| Temps de coag. (sec) | 58,8 | 59 | 50,4 | 45,4 |

TABLEAU 14

| Glycopeptide de l'invention (µg/ml) | 0 | 50 | 100 | 200 | 320 |
|---|---|---|---|---|---|
| Temps de coag. (sec) | 58 | 36 | 49,6 | 40,4 | 50 |

La reptilase n'est pas sensible à l'héparine. L'héparine n'inhibe la polymérisation de fibrinogène que par l'intermédiaire de la thrombine. Le glycopeptide de l'invention évaluéé à différente concentration ne provoque aucun allongement du temps de reptilase. Ceci est en faveur d'une activité anticoagulante ne mettant pas en jeu une dégradation ou une altération quelconque du fibrinogène par le glycopeptide de l'invention, son effet antipolymérisant s'exerçant surtout par une inhibition directe de la thrombine. Ceci est mis en évidence par le temps de fibrinogène dans un milieu non plasmatique et en absence d'anti-T III.

Conclusion

Les résultats présentés ci-dessus montrent que les mécanismes d'action de l'héparine et du glycopeptide de l'invention pour les facteurs étudiés sont différents, le glycopeptide de l'invention ayant comparativement une activité antithrombique directe plus importante que son activité catalytique. Le glycopeptide de l'invention, comme l'héparine, présente une totale inocuité vis-à-vis du fibrinogène.

Le tableau 15 ci-après est un tableau comparatif des propriétés de l'héparine et du glycopeptide de l'invention.

TABLEAU 15

Tableau recapitulatif de comparaison entre la myxaline et l'héparine

| | Myxaline | Heparine | Myxaline/heparine |
|---|---|---|---|
| 1. Activite antithrombique | 154 UI/mg | 173 UI/mg | 9% |
| Milieu plasmatique | 278 uNIH/mg | 3125 uNIH/mg | |
| Sur fibrinogène | 500 uNIH/mg | 1538 uNIH/mg | 32% |
| 2. Activite Anti Xa | 128 UantiXa/mg | 173 UantiXa/mg | 7% |
| 3. Temps de cephaline kaolin | | | |
| Double du temps Témoin | Héparine 42 fois plus active que la Myxaline | | |
| Temps | Héparine 16 fois plus active que la Myxaline | | |
| 4. Temps de QUICK | | | |
| Double du temps Témoin | Héparine 27 fois plus active que la Myxaline | | |
| Temps ∞ | Héparine 27 fois plus active que la Myxaline | | |

L'invention vise également toute composition contenant le glycopeptide selon l'invention, notamment toute composition présentant des propriétés anticoagulantes et contenant le glycopeptide selon l'invention.

L'invention vise plus particulièrement les compositions contenant le glycopeptide selon l'invention, lesquelles compositions ont les propriétés suivantes

leur poids moléculaire est compris d'environ 5 000 à environ 30 000, notamment à environ 20 000;

elles contiennent d'environ 50 à environ 55% (en poids) d'acides aminés, d'environ 5 à environ 10% (en poids) de motifs sucres et d'environ 0 à environ 4% (en poids) de lipides;

les motifs sucres contenus sont des hexoses et des hexoses aminés, appartenant au groupe constitué par la mannose, le glucose, le galactose, la galactosamine, la glucosamine;

leur comportement en chromatographie HPLC n'est pas modifié par le sodium dodécylsulfate;

elles sont anticoagulantes sur sang total à partir d'une concentration d'environ 1 600 µg/ml;

leur activité anticoagulante est d'environ 0,1 à environ 0,15 UI/mg;

elles entraînent un allongement du temps de Quick;

19

elles entraînent un allongement du temps de thrombine;
elles inactivent le facteur Xa.

Un exemple de composition contenant le glycopeptide de l'invention peut être tel que la fraction sucre contienne 30 à 40%, notamment 36% (en poids) de mannose, 20% à 30%, notamment 26% (en poids) de glucose, 15 à 25%, notamment 18% (en poids) de galactose, 5 à 15%, notamment 8% (en poids) de galactosamine, 5 à 15%, notamment 10% (en poids) de glucosamine.

Les résidus acides aminés peuvent être dosés par la méthode de Lowry.

Les sucres peuvent être dosés par la méthode à l'anthrone.

L'invention vise en particulier les compositions du type de l'extrait brut mentionné ci-dessus.

L'invention concerne en particulier les compositions dénommées ci-après extraits bruts, susceptibles d'être obtenues par la mise en oeuvre du procédé comprenant les étapes suivantes:

la séparation et la récupération du surnageant provenant d'un milieu de culture de myxobactéries du sous-ordre des cystobacterinae ou de mutantes de celles-ci;

l'élimination à partir du surnageant ainsi récupéré des substances de poids moléculaires inférieurs à environ 1 000 daltons;

l'élimination à partir du surnageant des protéines;

l'élimination à partir du surnageant des substances insolubles dans l'eau.

L'ordre dans lequel les trois étapes relatives respectivement à l'élimination des substances de poids moléculaires inférieurs à environ 1 000, des protéines et des produits insolubles dans l'eau n'intervient pas.

La matière première à partir de laquelle le glycopeptide de l'invention peut être obtenu peut être constituée par le surnageant provenant d'un milieu de culture de myxobactéries, du sous-ordre des Cystobacterinae ou de mutants de celles-ci.

Les Cystobacterinae se distinguent par le fait qu'elles produisent un mucus qui absorbe le rouge Congo.

Le milieu de culture de ces Cystobaterinae peut être constitué par un milieu solide ou liquide.

On a de préférence recours à un milieu de culture liquide.

La souche de myxobactéries utilisée est avantageusement constituée par une souche de Myxococcus xanthus.

La souche utilisée est par exemple la *Myxococcus xanthus* CM011, qui dérive de la souche I448, déposée le 3 mai 1985, par l'insertion (no. 11) d'un transposon Tn5 (selon la technique décrite dans "Introduction of transposon Tn5 into Myxococcus for analysis of developmental and other non selectable mutants", Proc. Natl. Acad. Sci. USA, 78, 425—429.

Cette insertion confère à la source la capacité de produire 50% de plus de sucres dans le milieu que la souche d'origine.

A titre d'exemple, on peut également utiliser la souche DK1622, telle que décrite dans Proc. Natl. Acad. Sci. USA, 76, 5 952—5 956, 1979.

Une telle souche est cultivée en milieu liquide, agité, constitué de Casitone Difco tamponné.

En ce qui concerne la séparation du surnageant, on prélève celui-ci de préférence au début de la phase stationnaire de croissance, en d'autres termes, à la fin de la phase exponentielle de croissance.

On a de préférence recours dans une première étape, à l'élimination des protéines, puis à l'élimination des substances de poids moléculaires inférieurs à environ 1 000 daltons, puis à l'élimination des substances insolubles dans l'eau.

L'élimination des protéines peut avoir lieu en utilisant du phénol.

Le phénol permet de faire précipiter les protéines et la phase aqueuse contient les compositions selon l'invention.

Le phénol utilisé est avantageusement du phénol 50% (poids/volume).

L'élimination des protéines peut également avoir lieu par filtration sur membrane, par exemple sur membrane de 10 000 daltons, notamment membrane de la marque Amicon 30D commercialisée par Amicon.

L'élimination des substances de poids moléculaires inférieurs à environ 1 000 peut être effectuée en utilisant de l'alcool éthylique pur.

L'addition d'alcool précipite les substances de poids moléculaires supérieurs à environ 1 000, tandis que les substances de poids moléculaires inférieurs à environ 1 000, se retrouvent dans l'alcool.

La phase de traitement à l'alcool a également pour but de concentrer.

Le précipité obtenu grâce à l'alcool peut être resolubilisé dans l'eau, la phase aqueuse obtenue étant retraitée par une nouvelle addition d'alcool, si l'on désire concentrer davantage.

On peut ensuite laver le précipité obtenu ci-dessus à l'acétone, pour délipider partiellement, puis à l'éther, notamment pour sécher.

On redissout le précipité dans de l'eau distillée.

L'élimination des produits insolubles peut être effectuée par centrifugation du précipité redissout.

Puis, on a avantageusement recours à un traitement de purification pour éliminer l'alcool ainsi que l'acétone et l'éther éventuellement utilisés dans les étapes précédentes.

Ce traitement de purification est avantageusement constitué par une ultrafiltration sur membrane, notamment 1 000 daltons; cette étape d'ultrafiltration est aussi une étape de concentration.

EP 0 219 400 B1

Cette étape de purification est avantageusement suivie d'une phase de concentration, par exemple sur membrane.

En ce qui concerne la phase d'élimination des insolubles, elle peut également avoir lieu après l'étape de purification et de concentration mentionnée ci-dessus.

Le produit brut selon l'invention peut notamment être conservé après lyophilisation ou évaporation sous vide.

Selon un mode de réalisation préféré, le procédé de l'invention comprend:

la récupération du surnageant provenant d'un milieu de culture de myxobactéries appartenant au groupe produisant un mucus colorable au rouge congo, laquelle culture est au début de la phase stationnaire de croissance,

l'addition au surnageant de phénol à 50% (poids/volume), ce qui conduit à l'obtention d'une phase aqueuse contenant la composition de l'invention et un précipité contenant les protéines;

la séparation des phases et la récupération de la phase aqueuse qui contient la composition selon l'invention,

l'addition d'alcool à la phase aqueuse débarrassée des protéines, ce qui conduit à l'obtention d'un précipité qui contient la composition selon l'invention, débarrassé des substances de poids moléculaires inférieurs à environ 1 000 daltons;

le lavage du susdit précipité à l'acétone, puis à l'éther, puis le séchage,

l'élimination des insolubles dans l'eau par dissolution du précipité obtenu ci-dessus, puis centrifugation,

la purification et la concentration de la phase obtenue ci-dessus par ultrafiltration,

la conservation de l'extrait brut par lyophilisation ou évaporation sous vide.

Selon un mode de réalisation avantageux, le procédé d'obtention des glycopeptides et des glycoprotéines de l'invention comprend en outre une étape dans laquelle on traite le surnageant afin d'éliminer les protéases, notamment par chauffage à environ 100°C, pendant un temps suffisant, par exemple environ 2 à environ 5 mn.

On donne ci-après le protocole détaillé de l'obtention d'une composition selon l'invention contenant le glycopeptide selon l'invention.

On prend la culture en début de phase stationnaire de croissance et on centrifuge à 8 000 g pendant 15 à 30 minutes pour éliminer les bactéries et récupérer le surnageant du milieu de culture.

On ajoute au surnageant un volume de phénol à 50% (poids/volume) dans l'eau distillée.

On agite énergiquement et on laisse agir à 60°C pendant 5 minutes.

On refroidit directement à 0°C dans un mélange réfrigérant (glace+eau) et on laisse reposer pour séparer la phase aqueuse du précipité qui s'est formé suite à l'addition de phénol.

On prélève la phase aqueuse qui contient la composition de l'invention, on centrifuge à 1 500 g à 0°C pendant 2 à 3 minutes et on récupère la phase aqueuse.

On effectue un second traitement au phénol 50%.

On précipite la composition de l'invention de la phase aqueuse en la traitant avec 5 volumes d'alcool 95° à −20°C pendant une nuit, ce qui permet ainsi d'éliminer le phénol et de concentrer.

On centrifuge à 3 000 g et à 0°C pendant 15 à 30 minutes.

On resuspend le précipité obtenu ci-dessus dans un petit volume d'eau distillée et on reprécipite avec 3 volumes d'alcool 95° à −20°C (comme précédemment), ce qui permet encore d'éliminer le phénol et de concentrer.

On lave à l'acétone, ce qui permet notamment de délipider partiellement, d'éliminer le phénol restant éventuellement, puis à l'éther, ce qui permet d'éliminer le phénol restant éventuellement (à −20°C) et on laisse sécher sur une paillasse à flux laminaire.

On redissout dans l'eau distillée.

On élimine les substances insolubles dans l'eau par centrifugation pendant 4—5 minutes à 160 g.

On lave les substances insolubles avec de l'eau distillée et on centrifuge comme précédemment.

On ultrafiltre notamment sur une membrane Amicon 1000D pour éliminer notamment l'alcool, l'acétone, l'éther et pour concentrer. On obtient la composition selon l'invention que l'on peut lyophiliser pour conserver.

En ce qui concerne les clelules utilisées, elles sont cultivées en milieu liquide (10 g/l de Bactocasitone commercialisé par Difco; 8 mM $MgSO_4$; Tris pH 7,6 10 mM; tampon phosphate pH 7,6 1 mM) à 30°C sous forte aération (agitation rotative à 300 rpm en erlen ou fermenteur de 7 l).

Selon un autre mode de réalisation préféré, le procédé d'obtention des compositions de l'invention correspond au procédé décrit ci-dessus dans lequel on remplace l'étape phénolique par une déprotéinisation partielle par ultrafiltration.

Résultats biologiques de la composition de l'invention obtenue par le procédé comportant la précipitation à l'aide de phénol

On donne ci-après les résultats biologiques de la composition de l'invention obtenue par le procédé ci-dessus décrit dans lequel on a recours à la précipitation à l'aide de phénol.

21

1. Coagulation sur sang total

Elle est effectuée selon le protocole indiqué à propos du glycopeptide. La concentration à partir de laquelle la composition obtenue par le procédé comportant la précipitation à l'aide de phénol est anticoagulante est de 1 600 µg/ml.

2. APTT ou temps de céphaline-kaolin

Les résultats relatifs au temps de céphaline-kaolin sont rassemblés dans le tableau 16 ci-après qui exprime le temps de céphaline-kaoline (en secondes) en fonction des concentrations différentes de la composition de l'invention (en µg/ml).

TABLEAU 16

| Témoin (tampon de Michaelis) Concentration de la composition dans le tampon (µg/ml) | Moyenne des temps de coagulation en secondes |
|---|---|
| | 44,7—46—45 |
| 600 | 35,2 |
| 1 000 | 19 |
| 1 500 | 10 |

Les résultats du tableau montrent qu'il y a réduction du temps de céphaline-kaolin.

3. Temps de quick

Les résultats sont donnés dans le tableau 17 dans lequel on a exprimé le temps de Quick pour des concentrations variables de la composition de l'invention.

TABLEAU 17

| Témoin (tampon de Michaelis) Concentration de la composition dans le tampon (µg/ml) | Moyenne des temps de coagulation en secondes |
|---|---|
| | 14,1—14,2—14 |
| 600 | 13,9 |
| 1 000 | 16,6 |
| 1 500 | 17,7 |

L'amélioration de la voie exogène montre que la composition de l'invention rallonge le temps de Quick et que cet effet est proportionnel à la concentration.

4. Temps de Xa

Les résultats concernant le temps de Xa sont rassemblés dans le tableau 18 ci-après qui exprime le temps de Xa pour des concentrations variables en composition de l'invention.

TABLEAU 18

| Témoin (tampon de Michaelis) Concentration de la composition (µg/ml) | Moyenne des temps de coagulation en secondes |
|---|---|
| | 18,1—17,9—18 |
| 600 | 19,8 |
| 1 000 | 23,6 |
| 1 500 | 33,3 |

D'après ce tableau, il résulte que la composition selon l'invention prolonge le temps de coagulation en inactivant le facteur Xa. Ainsi 1 000 µg/ml de composition inactivent 3,5 nanounités catalytiques de Xa.

5. Test de cytotoxicité

Ce test a été effectué in vitro sur des fibroblastes de poumons d'embryon de poulet à 14 jours d'incubation.

Le milieu de culture utilisé est le BME (base-médium-Eagel) contenant 2% de tricine, 2% de bicarbonate de sodium, et 10% de sérum de veau foetal (Eurobio).

Pour effectuer ce test, on utilise des boîtes de cultures Nunc (commercialisées par Polylabo) à 4 cupules.

Dans chaque cupule, on introduit le même nombre de cellules (environ 20 000 cellules/ml de milieu BME).

On incube à 37°C pendant 24 heures.

On rince doucement 2 ou 3 fois la culture avec le même milieu.

Dans deux cupules prises comme témoins, on introduit 1 ml de BME et dans les deux autres la composition de l'invention dissoute dans le même volume de milieu.

Différentes concentrations de polysaccharide ont été essayées: 20, 40, 60, 80 et 100 µg Geq/ml de BME, 1 µg Geq/ml représentant la quantité d'hexose de l'extrait.

Le comportement des cellules en présence du produit, en comparaison avec le témoin, suivi en microcinéma-vidéo à la température de 37°C (chambre de prise de vue thermostatée) a permis de montrer qu'aucun signe de cytotoxicité n'était constaté.

L'invention concerne également les compositions pharmaceutiques associant les susdits glycopeptides de l'invention et susdites compositions les contenant, avec un véhicule pharmaceutique.

Les glycopeptides de l'invention et les compositions les contenant sont particulièrement adaptés au contrôle (préventif ou curatif) de la coagulation sanguine chez l'homme ou l'animal, notamment dans ceux des cas où l'hôte est soumis à des risques d'hypercoagulabilité, plus particulièrement ceux résultant d'une perturbation de la phase extrinsèque susdite, par exemple comme conséquence d'une libération par l'organisme de thromboplastine, par exemple de thromboplastine tissulaire (interventions chirurgicales, processus athéromateux, développement de tumeurs, perturbations des mécanismes de la coagulation par des activateurs bactériens ou enzymatiques, etc.).

Les glycopeptides de l'invention et les compositions les contenant peuvent être sous forme de préparations administrables par voie orale ou rectale, en utilisant des solides ou des liquides appropriés pour un tel type d'administration ou sous forme de préparations injectables stériles contenant au moins l'une quelconque des compositions selon l'invention, en association avec des véhicules liquides appropriés stériles, de préférence isotoniques.

D'autres formes appropriées de préparations consistant en pommades dans lesquelles les glycopeptides selon l'invention et les compositions les contenant sont associés avec des véhicules en pommade.

On notera que les doses auxquelles les glycopeptides de l'invention et les compositions les contenant sont utilisés sont déterminées selon la nature de la maladie qui afflige le patient et les conditions particulières de santé.

Les dosages appropriés sont déterminés par le médecin, comme la pratique l'exige dans ces domaines d'application.

L'invention concerne également encore l'application des compositions selon l'invention à la constitution de réactifs biologiques utilisables au laboratoire, notamment à titre de référence de comparaison pour l'étude d'autres produits dont est testée l'activité anticoagulante.

**Revendications**

1. Glycopeptides et glycoprotéines présents dans le mucus excrété par les Myxobacteries Cystobacterinae ou dans le milieu de culture des Myxobacteries Cystobacterinae caractérisés par les propriétés suivantes:

leur poids moléculaire est compris d'environ 5 000 à environ 20 000 daltons, notamment d'environ 5 000 à environ 10 000;

les motifs sucres contenus sont des hexoses et des hexoses aminés, appartenant au groupe constitué par le mannose, le glucose, le galactose, la galactosamine, la glucosamine;

leur comportement en chromatographie HPLC n'est pas modifié par le sodium dodécylsulfate;

leur pH isoélectrique est d'environ 3 à environ 5, notamment d'environ 3,6;

ils sont anticoagulants sur sang total à partir d'une concentration d'environ 80 µg/ml;

leur activité anticoagulante est d'environ 2 à environ 10 UI/mg;

ils entraînent un allongement du temps de céphaline-kaolin;

ils entraînent un allongement du temps de Quick;

ils entraînent un allongement du temps de thrombine;

ils inactivent le facteur Xa.

2. Glycopeptides et glycoprotéines selon la revendication 1, caractérisés en ce qu'ils ont un poids moléculaire d'environ 6 300 daltons.

3. Glycopeptides et glycoprotéines selon la revendication 1, caractérisés en ce qu'ils ont la composition suivante:

70—80% (en poids) de résidus d'acides aminés;

30—20% (en poids) de motifs sucres, lesquels motifs sucres sont constitués par 60 à 65% (en poids) de sucres neutres et 35 à 40% (en poids) d'osamines.

4. Glycopeptides et glycoprotéines selon les revendications 1 à 2, caractérisés en ce que:

la composition des motifs sucres neutres est constituée par 30 à 35% (en poids) de glucose, 15 à 20% (en poids) de mannose, 10 à 15% (en poids) de galactose;

la composition des osamines est constituée de 20 à 25% (en poids) de glucosamine et de 10 à 15% (en poids) de galactosamine.

5. Glycopeptides et glycoprotéines selon la revendication 3, caractérisés en ce que la composition en sucres est

environ 35% (en poids) de glucose,

environ 11,4% (en poids) de galactose,

environ 16,3% (en poids) de mannose,

environ 23,3% (en poids) de glucosamine et

environ 14% (en poids) de galactosamine.

6. Glycopeptides et glycoprotéines selon les revendications 1 à 3, caractérisés en ce que la composition en acides aminés est la suivante:

30 à 35% (en poids) d'acide glutamique;

25 à 30% (en poids) de sérine;

5 à 10% (en poids) d'acide aspartique;

5 à 10% (en poids) de glycine;

5 à 10% (en poids) d'isoleucine;

5 à 10% (en poids) d'alanine;

0 à 5% (en poids) de valine;

0 à 5% (en poids) de leucine;

0 à 5% (en poids) de thréonine;

0 à 5% (en poids) de tyrosine;

moins de 2% (en poids) de proline;

moins de 2% (en poids) de lysine;

moins de 2% (en poids) de phénylalanine;

moins de 2% (en poids) d'histidine;

moins de 2% (en poids) d'arginine.

7. Glycopeptides et glycoprotéines selon la revendication 5, caractérisés en ce que la composition en acides aminés est la suivante:

environ 31% (en poids) d'acide glutamique,

environ 28% (en poids) de sérine,

environ 7% (en poids) d'acide aspartique,

environ 6% (en poids) de glycine,

environ 5,5% (en poids) d'isoleucine,

environ 5% (en poids) d'alanine,

environ 3% (en poids) de valine,

environ 3% de (en poids) leucine,

environ 2,5% (en poids) de thréonine,

environ 2,5% (en poids) de tyrosine,

moins d'environ 2% (en poids) de proline,

moins d'environ 2% (en poids) de phénylalanine,

moins d'environ 2% (en poids) d'histidine,

moins d'environ 2% (en poids) d'arginine.

8. Glycopeptides et glycoprotéines caractérisés en ce qu'ils sont obtenus par le procédé caractérisé par:

la séparation et la récupération du surnageant provenant d'un milieu de culture de myxobactéries du sous-ordre des Cystobacterinae ou de mutants de celles-ci;

l'élimination à partir du surnageant des substances de poids moléculaires inférieurs à environ 1 000 daltons;

l'élimination à partir du surnageant des substances insolubles dans l'eau;

et la récupération de la fraction du surnageant débarrassée des substances de poids moléculaires inférieurs à environ 1 000 daltons et débarrassée des substances insolubles dans l'eau;

la purification de cette fraction par chromatographie sur colonne de pseudo-affinité;

la purification subséquente de la susdite fraction par chromatographie sur colonne de gel de polymère réticulé de dextrane.

9. Glycopeptides et glycoprotéines caractérisés en ce qu'il sont obtenus par le procédé selon la revendication 8, dans lequel on traite le surnageant afin d'éliminer les protéases, par chauffage à environ 100°C, pendant environ 2 à environ 5 min.

10. Glycopeptides et glycoprotéines selon la revendication 8, caractérisés en ce que la souche utilisée est une souche de *Myxococcus xanthus* ou un mutant de celle-ci.

11. Glycopeptides et glycoprotéines selon la revendication 8, caractérisés en ce que la souche est la souche *Myxococcus xanthus* CM011, qui dérive de la souche déposée à la CNCM sous le numéro I448, par l'insertion d'un transposon Tn5.

12. Glycopeptides et glycoprotéines selon la revendication 8, caractérisés en ce qu'ils sont obtenus par le procédé comprenant:

la séparation du surnageant provenant d'un milieu de culture de myxobactéries du sous-ordre des Cystobacterinae ou de mutants de celles-ci, laquelle culture est au début de la phase stationnaire de croissance;

l'addition d'alcool au susdit surnageant, ce qui conduit à l'obtention d'un précipité qui contient les glycopeptides selon l'invention et qui est débarrassé des substances de poids moléculaires inférieurs à environ 1 000 daltons;

le lavage du susdit précipité à l'acétone, puis à l'éther, puis le séchage du précipité;

la dissolution dans l'eau distillée du susdit précipité séché;

l'élimination des substances insolubles dans l'eau, notamment par centrifugation et l'obtention d'une solution contenant les glycopeptides de l'invention et débarrassée des substances de poids moléculaires inférieurs à environ 1 000 daltons, et des substances insolubles dans l'eau;

la purification par ultrafiltration et la concentration de la solution obtenue ci-dessus, ce qui conduit à l'obtention d'une fraction contenant les glycopeptides;

la conservation de la susdite fraction par lyophilisation ou évaporation sous vide;

la purification par chromatographie sur colonne de pseudo-affinité de la susdite fraction;

la purification subséquente par chromatographie sur colonne de gel de polymère réticulé de dextrane.

13. Glycopeptides et glycoprotéines selon la revendication 8, caractérisés par le procédé comprenant:

la séparation et la récupération du surnageant provenant d'un milieu de cultures de myxobactéries du sous-ordre des Cystobacterinae ou de mutants de celles-ci;

l'élimination à partir du surnageant ainsi récupéré:

des protéines,

des substances de poids moléculaires inférieurs à environ 1 000 daltons,

des substances insolubles dans l'eau;

pour donner un extrait brut;

la purification par chromatographie sur colonne de pseudo-affinité du susdit extrait brut;

la purification subséquente par chromatographie sur colonne de gel de polymère réticulé de dextrane.

14. Glycopeptides et glycoprotéines selon la revendication 12, caractérisés par le procédé comprenant:

la séparation et la récupération du surnageant provenant d'un milieu de cultures de myxobactéries appartenant au groupe produisant un mucus colorable au rouge Congo, laquelle culture est au début de la phase stationnaire de croissance;

l'addition au surnageant de phénol à 50% (poids/volume), qui conduit à:

l'obtention d'un précipité contenant les protéines;

et à l'obtention d'une phase aqueuse contenant les glycopeptides selon l'invention;

la séparation des phases et la récupération de la phase aqueuse qui contient les glycopeptides selon l'invention;

l'addition d'alcool à la susdite phase aqueuse ce qui conduit à l'obtention d'un précipité qui contient les glycopeptides selon l'invention et qui est débarrassé des protéines et des substances de poids moléculaires inférieurs à environ 1 000 daltons;

le lavage du susdit précipité à l'acétone, puis à l'éther, puis le séchage du précipité;

la dissolution dans l'eau distillée du susdit précipité séché;

l'élimination des substances insolubles dans l'eau, notamment par centrifugation et l'obtention d'une solution contenant les glycopeptides de l'invention et débarrassée des substances de poids moléculaires inférieurs à environ 1 000 daltons, des protéines et des substances insolubles dans l'eau;

la purification par ultrafiltration et la concentration de la solution obtenue ci-dessus, ce qui conduit à l'obtention d'un extrait brut contenant les glycopeptides;

la conservation du susdit extrait brut par lyophilisation ou évaporation sous vide;

la purification par chromatographie sur colonne de pseudo-affinité de l'extrait brut;

la purification subséquente par chromatographie sur colonne de gel de polymère réticulé de dextrane.

15. Glycopeptides et glycoprotéines caractérisés en ce qu'ils sont obtenus par le procédé selon la revendication 14, dans lequel on traite le surnageant afin d'éliminer les protéases, notamment par chauffage à 100°C, pendant 2 à 5 min.

16. Mélange contenant des glycopeptides et glycoprotéines selon les revendications 1 à 14, caractérisée par les propriétés suivantes:

son poids moléculaire est compris d'environ 5 000 à environ 30 000, notamment à environ 20 000;

elle contient d'environ 50 à environ 55% (en poids) d'acides aminés, d'environ 5 à environ 10% (en poids), de motifs sucres et d'environ 3 à environ 4% (en poids) de lipides;

les motifs sucres contenus sont des hexoses et des hexoses aminés, appartenant au groupe constitué par la mannose, le glucose, le galactose, la galactosamine, la glucosamine;

son comportement en chromatographie HPLC n'est pas modifié par le sodium dodécylsulfate;

elle est anticoagulant sur sang total à partir d'une concentration d'environ 1 600 µg/ml;

son activité anticoagulante est d'environ 0,1 à 0,15 UI/mg;

elle entraîne un allongement du temps de Quick;

elle entraîne un allongement du temps de thrombine;

elle inactive le facteur Xa.

17. Composition selon la revendication 16, caractérisé en ce que la composition en motifs sucres est constituée par 30 à 40%, notamment 36% (en poids) de mannose, 20% à 30%, notamment 26% (en poids) de glucose, 15 à 25%, notamment 18% (en poids) de galactose, 5 à 15%, notamment 8% (en poids) de galactosamine, 5 à 15%, notamment 10% (en poids) de glucosamine.

18. Composition selon la revendication 16, caractérisée en ce qu'elle est obtenue par le procédé comprenant les étapes suivantes:

la récupération du surnageant provenant d'un milieu de culture de myxobactéries appartenant au groupe produisant un mucus colorable au rouge congo, laquelle culture est au début de la phase stationnaire de croissance,

l'addition au surnageant de phénol à 50% (poids/volume), ce qui conduit à l'obtention d'une phase aqueuse contenant la composition de l'invention et un précipité contenant les protéines;

la séparation des phases et la récupération de la phase aqueuse qui contient la composition selon l'invention,

l'addition d'alcool à la phase aqueuse débarrassée des protéines, ce qui conduit à l'obtention d'un précipité qui contient la composition selon l'invention, débarrassé des substances de poids moléculaires inférieures à environ 1 000 daltons;

le lavage du susdit précipité à l'acétone, puis à l'éther, puis le séchage,

l'élimination des insolubles dans l'eau par dissolution du précipité obtenu ci-dessus, puis centrifugation,

la purification et la concentration de la phase obtenue ci-dessus par ultrafiltration,

la conservation de l'extrait brut par lyophilisation ou évaporation sous vide.

19. Composition caractérisée en ce qu'elle est obtenue par le procédé selon la revendication 18, dans lequel on traite le surnageant afin d'éliminer les protéases notamment par chauffage à 100°C, pendant 2 à 5 min.

**Patentansprüche**

1. Glycopeptide und Glycoproteine, die in dem von den Myxobakterien Cystobacterinae ausgeschiedenen Schleim oder im Kulturmilieu der Myxobakterien Cystobacterinae vorhanden sind, gekennzeichnet durch die folgenden Eigenschaften:

ihr Molekulargewicht umfaßt von ungefähr 5 000 bis ungefähr 20 000 Dalton, insbesondere von ungefähr 5 000 bis ungefähr 10 000;

die enthaltenen Zuckerbestandteile sind Hexosen und Hexosamine, die zur Gruppe bestehend aus Mannose, Glucose, Galactose, Galactosamin, Glucosamin gehören;

ihr Verhalten bei HPLC-Chromatographie wird von Natrium-Dodecylsulfat nicht verändert;

ihr isoelektrischer pH ist von ungefähr 3 bis ungefähr 5, insbesondere ungefähr 3,6;

sie sind Antikoagulantien für Vollblut ab einer Konzentration von ungefähr 80 µg/ml;

ihre antikoagulative Aktivität ist von ungefähr 2 bis ungefähr 10 UI/mg;

sie haben eine Zeitverlängerung von Cephalin-Kaolin zur Folge;

sie haben eine Zeitverlängerung von Quick zur Folge;

sie haben eine Zeitverlängerung von Thrombin zur Folge;

sie inaktivieren den Faktor Xa.

2. Glycopeptide und Glycoproteine gemäß Anspruch 1, dadurch gekennzeichnet, daß sie ein Molekulargewicht von ungefähr 6 300 Dalton haben.

3. Glycopeptide und Glycoproteine gemäß Anspruch 1, dadurch gekennzeichnet, daß sie folgende Zusammensetzung haben:

70—80 Gewichts-% Aminosäurereste;

30—20 Gewichts-% Zuckerbestandteile, wobei diese Zuckerbestandteile aus 60 bis 65 Gewichts-% neutralen Zuckern und 35 bis 40 Gewichts-% Osaminen bestehen.

4. Glycopeptide und Glycoproteine gemäß den Ansprüchen 1 bis 2, dadurch gekennzeichnet, daß:

die Zusammensetzung der neutralen Zuckerbestandteile besteht aus 30 bis 35 Gewichts-% Glucose, 15 bis 20 Gewichts-% Mannose und 10 bis 15 Gewichts-% Galactose;

die Zusammensetzung der Osamine besteht aus 20 bis 25 Gewichts-% Glucosamin und von 10 bis 15 Gewichts-% Galactosamin.

5. Glycopeptide und Glycoproteine gemäß Anspruch 3, dadurch gekennzeichnet, daß die Zusammensetzung der Zucker ist

ungefähr 35 Gewichts-% Glucose,

ungefähr 11,4 Gewichts-% Galactose,

ungefähr 16,3 Gewichts-% Mannose,

ungefähr 23,3 Gewichts-% Glucosamin und

ungefähr 14 Gewichts-% Galactosamin.

6. Glycopeptide und Glycoproteine gemäß den Ansprüchen 1 bis 3, dadurch gekennzeichnet, daß die Zusammensetzung der aminierten Säuren folgende ist:

30 bis 35 Gewichts-% Glutaminsäure;
25 bis 30 Gewichts-% Serin;
5 bis 10 Gewichts-% Asparaginsäure;
5 bis 10 Gewichts-% Glycin;
5 bis 10 Gewichts-% Isoleucin;
5 bis 10 Gewichts-% Alanin;
0 bis 5 Gewichts-% Valin;
0 bis 5 Gewichts-% Leucin;
0 bis 5 Gewichts-% Threonin;
0 bis 5 Gewichts-% Tyrosin;
weniger als 2 Gewichts-% Prolin;
weniger als 2 Gewichts-% Lysin;
weniger als 2 Gewichts-% Phenylalanin;
weniger als 2 Gewichts-% Histidin;
weniger als 2 Gewichts-% Arginin.

7. Glycopeptide und Glycoproteine gemäß Anspruch 5, dadurch gekennzeichnet, daß die Zusammensetzung der aminierten Säuren folgende ist:

ungefähr 31 Gewichts-% Glutaminsäure,
ungefähr 28 Gewichts-% Serin,
ungefähr 7 Gewichts-% Asparaginsäure,
ungefähr 6 Gewichts-% Glycin,
ungefähr 5,5 Gewichts-% Isoleucin,
ungefähr 5 Gewichts-% Alanin,
ungefähr 3 Gewichts-% Valin,
ungefähr 3 Gewichts-% Leucin,
ungefähr 2,5 Gewichts-% Threonin,
ungefähr 2,5 Gewichts-% Tyrosin,
weniger als ungefähr 2 Gewichts-% Prolin,
weniger als ungefähr 2 Gewichts-% Phenylalanin,
weniger als ungefähr 2 Gewichts-% Histidin,
weniger als ungefähr 2 Gewichts-% Arginin.

8. Glycopeptide und Glycoproteine erhalten durch ein Verfahren gekennzeichnet durch:

das Abtrennen und Gewinnen des Überstandes, der von einem Kulturmilieu von Myxobakterien der Unterordnung Cystobacterinae oder von Mutanten davon herstammt;

die Eliminierung ausgehend vom Überstand von Substanzen mit Molekulargewichten unter ungefähr 1 000 Dalton;

die Eliminierung ausgehend vom Überstand von Substanzen, die in Wasser unlöslich sind;

und das Gewinnen der Fraktion des Überstandes, die frei ist von Substanzen mit Molekulargewichten unter ungefähr 1 000 Dalton und frei ist von Substanzen, die in Wasser unlöslich sind;

die Reinigung dieser Fraktion durch Chromatographie auf einer pseudo-affinen Kolonne;

die nachfolgende Reinigung der genannten Fraktion durch Chromatographie auf einer Kolonne mit vernetztem Dextran-Polymergel.

9. Glycopeptide und Glycoproteine dadurch gekennzeichnet, daß sie durch das Verfahren gemäß Anspruch 8 erhalten werden, in welchem der Überstand behandelt wird, um die Proteasen zu eliminieren, durch Erhitzen auf ungefähr 100°C während ungefähr 2 bis ungefähr 5 min.

10. Glycopeptide und Glycoproteine gemäß Anspruch 8, dadurch gekennzeichnet, daß der verwendete Stamm ein Stamm von *Myxococcus xanthus* oder eines Mutanten davon ist.

11. Glycopeptide und Glycoproteine gemäß Anspruch 8, dadurch gekennzeichnet, daß der Stamm der Stamm *Myxococcus xanthus* CM011 ist, der durch Einführen eines Transposons Tn5 von dem Stamm abgeleitet ist, der bei der CNCM unter der Nummer I448 hinterlegt ist.

12. Glycopeptide und Glycoproteine gemäß Anspruch 8, dadurch gekennzeichnet, daß sie erhalten werden nach dem Verfahren, das umfaßt:

das Abtrennen des Überstandes, der von einem Kulturmilieu von Myxobakterien der Unterordnung Cystobacterinae oder von Mutanten davon herstammt, wobei diese Kultur am Beginn der stationären Phase des Wachstums ist;

die Zugabe von Alkohol zum genannten Überstand, was zum Erhalt eines Niederschlags führt, der die erfindungsgemäßen Glycopeptide enthält und der frei ist von Substanzen mit Molekulargewichten unter ungefähr 1 000 Dalton;

das Waschen des genannten Niederschlags mit Aceton, dann mit Ether, dann das Trocknen des Niederschlags;

das Auflösen des genannten getrockneten Niederschlags in destilliertem Wasser;

EP 0 219 400 B1

die Eliminierung der in Wasser unlöslichen Substanzen, insbesondere durch Zentrifugieren und der Erhalt einer Lösung, die die Glycopeptide der Erfindung enthält und frei ist von Substanzen mit Molekulargewichten unter ungefähr 1 000 Dalton und von Substanzen, die in Wasser unlöslich sind;

die Reinigung durch Ultrafiltration und die Konzentrierung der dadurch erhaltenen Lösung, was zum Erhalt einer Fraktion führt, die die Glycopeptide enthält;

die Konservierung der genannten Fraktion durch Lyophilisierung oder Verdampfung unter Vakuum;

die Reinigung der genannten Fraktion durch Chromatographie über eine pseudo-affine Kolonne;

die nachfolgende Reinigung durch Chromatographie über eine Kolonne mit vernetztem Dextran-Polymergel.

13. Glycopeptide und Glycoproteine gemäß Anspruch 8, gekennzeichnet durch das Verfahren, das umfaßt:

das Abtrennen und das Aufnehmen des Überstandes, der von einem Kulturmilieu von Myxobakterien der Unterordnung Cystobacterinae oder von Mutanten davon herstammt;

die Eliminierung ausgehend vom so aufgenommenen Überstand:

von Proteinen,

von Substanzen mit Molekulargewichten unter

ungefähr 1 000 Dalton,

von Substanzen, die in Wasser unlöslich sind;

um einen Rohextrakt zu erhalten;

die Reinigung des genannten Rohextrakts durch Chromatographie über eine pseudo-affine Kolonne;

die nachfolgende Reinigung durch Chromatographie über eine Kolonne mit vernetztem Dextran-Polymergel.

14. Glycopeptide und Glycoproteine gemäß Anspruch 12, gekennzeichnet durch das Verfahren, das umfaßt:

das Abtrennen und das Aufnehmen des Überstandes, der von einem Kulturmilieu von Myxobakterien, die zur Gruppe gehören, die einen mit Kongorot anfärbbaren Schleim produzieren, diese Kultur ist am Beginn der stationären Phase des Wachstums;

die Zugabe von Phenol bis 50% (Gewicht/Volumen) zum Überstand, was bewirkt:

den Erhalt eines Niederschlags, der die Proteine enthält;

und den Erhalt einer wässrigen Phase, die die erfindungsgemäßen Glycopeptide enthält;

das Trennen der Phasen und das Aufnehmen der wässrigen Phase, die die erfindungsgemäßen Glycopeptide enthält;

die Zugabe von Alkohol zur genannten wässrigen Phase, was zum Erhalt eines Niederschlags führt, der die erfindungsgemäßen Glycopeptide enthält und der frei ist von Proteinen und von Substanzen mit Molekulargewichten unter ungefähr 1 000 Dalton;

das Waschen des genannten Niederschlags mit Aceton, dann mit Ether, dann das Trocknen des Niederschlags;

das Auflösen des genannten getrockneten Niederschlags in destilliertem Wasser;

die Eliminierung von in Wasser unlöslichen Substanzen, insbesondere durch Zentrifugieren und der Erhalt einer Lösung, die die Glycopeptide der Erfindung enthält und frei ist von Substanzen mit Molekulargewichten unter ungefähr 1 000 Dalton, von Proteinen und von in Wasser unlöslichen Substanzen;

die Reinigung durch Ultrafiltration und die Konzentrierung der dadurch erhaltenen Lösung, was zum Erhalt eines Rohextrakts führt, der die Glycopeptide enthält;

die Konservierung des genannten Rohextrakts durch Lyophilisierung oder Verdampfung unter Vakuum;

die Reinigung des Rohextrakts durch Chromatographie über eine pseudo-affine Kolonne;

die nachfolgende Reinigung durch Chromatographie über eine Kolonne mit vernetztem Dextran-Polymergel.

15. Glycopeptide und Glycoproteine dadurch gekennzeichnet, daß sie nach dem Verfahren gemäß Anspruch 14 erhalten werden, in welchem der Überstand behandelt wird, um die Proteasen zu eliminieren, insbesondere durch Erhitzen auf 100°C, während 2 bis 5 min.

16. Mischung, die die Glycopeptide und Glycoproteine gemäß den Ansprüchen 1 bis 14 enthält, gekennzeichnet durch die folgenden Eigenschaften:

ihr Molekulargewicht umfaßt von ungefähr 5 000 bis ungefähr 30 000, insbesondere bis ungefähr 20 000;

sie enthält von ungefähr 50 bis ungefähr 55 Gewichts-% Amino-Säuren, von ungefähr 5 bis ungefähr 10 Gewichts-% Zuckerbestandteile und von ungefähr 3 bis ungefähr 4 Gewichts-% Lipide;

die enthaltenen Zuckerbestandteile sind Hexosen und Hexosamine, die zur Gruppe bestehend aus Mannose, Glucose, Galactose, Galactosamin, Glucosamin gehören;

ihr Verhalten bei HPLC-Chromatographie wird von Natrium-Dodecylsulfat nicht verändert;

sie ist ein Antikoagulans für Vollblut ab einer Konzentration von ungefähr 1 600 µg/ml;

ihre antikoagulative Aktivität ist von ungefähr 0,1 bis ungefähr 0,15 UI/mg;

sie hat eine Zeitverlängerung von Quick zur Folge;

sie hat eine Zeitverlängerung von Thrombin zur Folge;

sie inaktiviert den Faktor Xa.

17. Zusammensetzung gemäß Anspruch 16, dadurch gekennzeichnet, daß die Zusammensetzung an Zuckerbestandteilen besteht aus 30 bis 40 Gewichts-%, insbesondere 36 Gewichts-% Mannose, 20 bis 30 Gewichts-%, insbesondere 26 Gewichts-% Glucose, 15 bis 25 Gewichts-%, insbesondere 18 Gewichts-% Galactose, 5 bis 15 Gewichts-%, insbesondere 8 Gewichts-% Galactosamin, 5 bis 15 Gewichts-%, insbesondere 10 Gewichts-% Glucosamin.

18. Zusammensetzung gemäß Anspruch 16, dadurch gekennzeichnet, daß sie durch das Verfahren erhalten wird, das folgende Stufen umfaßt:

das Aufnehmen des Überstandes, der von einem Kulturmilieu von Myxobakterien herstammt, die zur Gruppe gehören, die einen mit Kongorot anfärbbaren Schleim produzieren, diese Kultur ist am Beginn der stationären Phase des Wachstums;

die Zugabe von Phenol bis 50% (Gewicht/Volumen) zum Überstand, was zum Erhalt einer wässrigen Phase führt, die die Zusammensetzung der Erfindung enthält und einen Niederschlag, der die Proteine enthält;

das Trennen der Phasen und das Aufnehmen der wässrigen Phase, die die erfindungsgemäße Zusammensetzung enthält;

die Zugabe von Alkohol zur wässrigen Phase, die von Proteinen frei ist, was zum Erhalt eines Niederschlags führt, der die erfindungsgemäße Zusammensetzung enthält, frei von Substanzen mit Molekulargewichten unter ungefähr 1 000 Dalton;

das Waschen des genannten Niederschlags mit Aceton, dann mit Ether, dann das Trocknen;

die Eliminierung des in Wasser Unlöslichen durch Lösen des dadurch erhaltenen Niederschlags, dann Zentrifugieren,

die Reinigung und die Konzentrierung der dadurch erhaltenen Phase durch Ultrafiltration,

die Konservierung des Rohextrakts durch Lyophilisierung oder Verdampfung unter Vakuum.

19. Zusammensetzung dadurch gekennzeichnet, daß sie durch das Verfahren gemäß Anspruch 18 erhalten wird, in dem der Überstand behandelt wird, um die Proteasen zu eliminieren, insbesondere durch Erhitzen auf 100°C, für 2 bis 5 min.

**Claims**

1. Glycopeptides and glycoproteins present in the mucus excreted by the myxobacteria Cystobacterinae or into the culture medium of the myxobacteria Cystobacterinae characterized by the following properties:

their molecular weight is included between about 5,000 and about 20,000, in particular between about 5,000 and about 10,000, daltons;

the sugar residues contained are hexoses and aminated hexoses, belonging to the group constituted by mannose, glucose, galactose, galactosamine, glucosamine;

their behaviour on HPLC chromatography is not modified by sodium dodecylsulfate;

their isoelectric pH is about 3 to about 5, in particular about 3.6;

they are anticoagulants for whole blood from a concentration of about 80 µg/ml;

their anticoagulant activity is about 2 to about 10 IU/mg;

they lead to a lengthening of the kaolin-cephaline thromboplastin time;

they lead to a lengthening of the Quick time;

they lead to a lengthening of the thrombin time;

they inactivate the factor Xa.

2. Glycopeptides and glycoproteins according to Claim 1, characterized in that they have a molecular weight of about 6,300 daltons.

3. Glycopeptides and glycoproteins according to Claim 1, characterized in that they have the following composition:

70—80% (by weight) of amino acid residues;

30—20% (by weight) of sugar residues these sugar residues are constituted by 60—65% (by weight) of neutral sugars and 35—40% (by weight) of amino sugars.

4. Glycopeptides and glycoproteins according to the Claims 1 to 2, characterized in that:

the composition of the neutral sugar residues is constituted by 30 to 35% (by weight) of glucose, 15 to 20% (by weight) of mannose, 10 to 15% (by weight) of galactose;

the composition of the amino sugars is constituted of 20 to 25% (by weight) of glucosamine and of 10 to 15% (by weight) of galactosamine.

5. Glycopeptides and glycoproteins according to Claim 3, characterized in that the composition of the sugars is

about 35% (by weight) of glucose,

about 11.4% (by weight) of galactose,

about 16.3% (by weight) of mannose,

about 23.3% (by weight) of glucosamine and

about 14% (by weight) of galactosamine.

29

6. Glycopeptides and glycoproteins according to Claims 1 to 3, characterized in that the composition of the amino acids is the following:

30 to 35% (by weight) of glutamic acid;
25 to 30% (by weight) of serine;
5 to 10% (by weight) of aspartic acid;
5 to 10% (by weight) of glycine;
5 to 10% (by weight) of isoleucine;
5 to 10% (by weight) of alanine;
0 to 5% (by weight) of valine;
0 to 5% (by weight) of leucine;
0 to 5% (by weight) of threonine;
0 to 5% (by weight) of tyrosine;
less than 2% (by weight) of proline;
less than 2% (by weight) of lysine;
less than 2% (by weight) of phenylalanine;
less than 2% (by weight) of histidine;
less than 2% (by weight) of arginine.

7. Glycopeptides and glycoproteins according to Claim 5, characterized in that the composition of the amino acids is the following:

about 31% (by weight) of glutamic acid,
about 28% (by weight) of serine,
about 7% (by weight) of aspartic acid,
about 6% (by weight) of glycine,
about 5.5% (by weight) of isoleucine,
about 5% (by weight) of alanine,
about 3% (by weight) of valine,
about 3% (by weight) of leucine,
about 2.5% (by weight) of threonine,
about 2.5% (by weight) of tyrosine,
less than about 2% (by weight) of proline,
less than about 2% (by weight) of phenylalanine,
less than about 2% (by weight) of histidine,
less than about 2% (by weight) of arginine.

8. Glycopeptides and glycoproteins characterized in that they are obtained by means of the procedure characterized by:

the separation and the recovery of the supernatant derived from a culture medium of myxobacteria of the sub-order of the Cystobacterinae or mutants of these latter;

the removal from the supernatant of the substances of molecular weight less than about 1,000 daltons;

the removal from the supernatant of the substances insoluble in water;

the recovery of the fraction of the supernatant freed of the substances of molecular weight less than about 1,000 daltons and freed of the substances insoluble in water;

the purification of this fraction by chromatography on a pseudo-affinity column;

the subsequent purification of the above-mentioned fraction by chromatography on a column of a cross-linked dextran polymer gel.

9. Glycopeptides and glycoproteins characterized in that they are obtained by the procedure according to Claim 8, in which the supernatant is treated by heating at about 100°C for about 2 to about 5 min. in order to destroy the proteases.

10. Glycopeptides and glycoproteins according to Claim 8, characterized in that the strain used is a strain of *Myxococcus xanthus* or a mutant of this latter.

11. Glycopeptides and glycoproteins according to Claim 8, characterized in that the strain is the *Myxococcus xanthus* CM011 strain, which derives from the strain deposited with the NCCM under the number I-448 by the insertion of a transposon Tn5.

12. Glycopeptides and glycoproteins according to Claim 8, characterized in that they are obtained by the procedure comprising:

the separation of the supernatant derived from a culture medium of myxobacteria of the sub-order of the Cystobaterinae or mutants of these latter, which culture is at the beginning of the stationary phase of growth;

the addition of alcohol to the above-mentioned supernatant, which leads to the formation of a precipitate which contains the glycopeptides according to the invention and which is freed of substances of molecular weight lower than about 1,000 daltons;

the washing of the above-mentioned precipitate with acetone, then with ether, followed by drying of the precipitate;

the dissolution of the above-mentioned dried precipitate in distilled water;

the removal of the substances insoluble in water, in particular by centrifugation and the recovery of a solution containing the glycopeptides of the invention freed of the substances of molecular weight lower than about 1,000 daltons and the substances insoluble in water;

the purification by ultrafiltration and concentration of the solution obtained above, which leads to the preparation of a fraction containing the glycopeptides;

the preservation of the above-mentioned fraction by lyophilisation or evaporation in a vacuum;

the purification of the above-mentioned fraction by chromatography on a pseudo-affinity column;

the subsequent purification by chromatography on a column of cross-linked dextran polymer gel.

13. Glycopeptides and glycoproteins according to Claim 8, characterized by the procedure comprising:

the separation and recovery of the supernatant derived from a culture medium of myxobacteria of the sub-order of the Cystobacterinae or mutants of these latter;

the removal from the supernatant thus recovered of:

proteins,

substances of molecular weight less than about 1,000 daltons,

substances insoluble in water;

in order to give a crude extract;

the purification of the above-mentioned crude extract by chromatography on a pseudo-affinity column;

the subsequent purification by chromatography on a colum of a cross-linked dextran polymer gel.

14. Glycopeptides and glycoproteins according to Claim 12, characterized by the procedure comprising:

the separation and the recovery of the supernatant derived from a culture medium of myxobacteria belonging to the group producing a mucus stainable by Congo red, which culture is at the beginning of the stationary phase of growth;

the addition of phenol to the supernatant to 50% (weight/volume), which leads to:

the formation of a precipitate containing the proteins;

and the formation of an aqueous phase containing the glycopeptides according to the invention;

the separation of the phases and the recovery of the aqueous phase which contains the glycopeptides according to the invention;

the addition of alcohol to the above-mentioned aqueous phase which leads to the formation of a precipitate which contains the glycopeptides according to the invention and which is freed of the proteins and of the substances of molecular weight lower than about 1,000 daltons;

the washing of the above-mentioned precipitate with acetone, then with ether, followed by drying of the precipitate;

the dissolution of the above-mentioned dried precipitate in distilled water;

the removal of the substances insoluble in water, in particular by centrifugation and the obtaining of a solution containing the glycopeptides of the invention freed of the substances of molecular weight lower than about 1,000 daltons, the proteins and the substances insoluble in water;

the purification by ultrafiltration and concentration of the solution obtained above, which leads to the preparation of a crude extract containing the glycopeptides;

the preservation of the above-mentioned crude extract by lyophilisation or evaporation in a vacuum;

the purification of the crude extract by chromatography on a pseudo-affinity column;

the subsequent purification by chromatography on a column of cross-linked dextran polymer gel.

15. Glycopeptides and glycoproteins characterized in that they are obtained by the procedure according to Claim 14, in which the supernatant is treated in particular by heating to 100°C for 2 to 5 min in order to destroy the proteases.

16. Mixture containing glycopeptides and glycoproteins according to Claims 1 to 14, characterized by the following properties:

its molecular weight is included between about 5,000 and about 30,000, and is in particular about 20,000;

it contains from about 50 to about 55% (by weight) of amino acids, about 5 to about 10% (by weight) of sugar residues and about 3 to about 4% (by weight) of lipids;

the sugar residues contained are hexoses and amino hexoses, belonging to the group constituted by mannose, glucose, galactose, galactosamine, glucosamine;

its behaviour on HPLC chromatography is not modified by sodium dodecylsulfate;

it is anticoagulant for whole blood from a concentration of about 1,600 µg/ml;

its anticoagulant activity is about 0.1 to 0.15 IU/mg;

it leads to a lengthening of the Quick time;

it leads to a lengthening of the thrombin time;

it inactivates the factor Xa.

17. Composition according to Claim 16, characterized in that the composition of the sugar residues is constituted by 30 to 40%, in particular 36% (by weight) of mannose, 20 to 30%, in particular 26% (by weight) of glucose, 15 to 25%, in particular 18% (by weight) of galactose, 5 to 15%, in particular 8% (by weight) of galactosamine, 5 to 15%, in particular 10% (by weight) of glucosamine.

18. Composition according to Claim 16, characterized in that it is obtained by the procedure comprising the following steps:

the recovery of the supernatant derived from a culture medium of myxobacteria belonging to the

**EP 0 219 400 B1**

group producing a mucus stainable by Congo red, which culture is at the beginning of the stationary phase of growth,

the addition of phenol to the supernatant to 50% (weight/volume), which leads to the formation of an aqueous phase containing the composition of the invention and a precipitate containing the proteins;

the separation of the phases and the recovery of the aqueous phase which contains the composition according to the invention,

the addition of alcohol to the aqueous phase freed of proteins, which leads to the formation of a precipitate which contains the composition according to the invention, freed of the substances of molecular weight lower than about 1,000 daltons;

the washing of the above-mentioned precipitate with acetone, then with ether, followed by drying,

the removal of the insolubles in water by dissolution of the precipitate obtained above, followed by centrifugation;

the purification and the concentration of the phase obtained above by ultrafiltration,

the preservation of the crude extract by lyophilisation or evaporation in a vacuum.

19. Composition characterized in that it is obtained by the procedure according to Claim 18, in which the supernatant is treated by heating at 100°C for 2 to 5 min in order to destroy the proteases.

32

FIG.1

FIG.2

FIG.3

EP 0 219 400 B1

FIG.4

FIG.5A

FIG.5B

FIG.6

FIG.7

4